(19) Europäisches Patentamt
European Patent Office
Office européen des brevets

(11) **EP 3 397 336 B1**

(12) **EUROPÄISCHE PATENTSCHRIFT**

(45) Veröffentlichungstag und Bekanntmachung des Hinweises auf die Patenterteilung:
**01.07.2020 Patentblatt 2020/27**

(51) Int Cl.:
*A61N 5/067* (2006.01)   *A61N 1/05* (2006.01)
*A61N 1/36* (2006.01)   *A61N 5/06* (2006.01)

(21) Anmeldenummer: **16826075.0**

(22) Anmeldetag: **29.12.2016**

(86) Internationale Anmeldenummer:
**PCT/EP2016/082797**

(87) Internationale Veröffentlichungsnummer:
**WO 2017/114878 (06.07.2017 Gazette 2017/27)**

(54) **VORRICHTUNG ZUR EFFEKTIVEN INVASIVEN MEHRSEGMENT-NEUROSTIMULATION**

DEVICE FOR AN EFFECTIVE INVASIVE MULTI-SEGMENT NEUROSTIMULATION

DISPOSITIF POUR LA NEUROSTIMULATION NEUROLOGIQUE INVASIVE EFFICACE À PLUSIEURS SEGMENTS

(84) Benannte Vertragsstaaten:
**AL AT BE BG CH CY CZ DK EE ES FI FR GB GR HR HU IE IS IT LI LT LU LV MC MK MT NL NO PL PT RO RS SE SI SK SM TR**

(30) Priorität: **29.12.2015 DE 102015122888**

(43) Veröffentlichungstag der Anmeldung:
**07.11.2018 Patentblatt 2018/45**

(73) Patentinhaber: **Forschungszentrum Jülich GmbH 52425 Jülich (DE)**

(72) Erfinder:
• **TASS, Peter, Alexander**
  **83684 Tegernsee (DE)**
• **HALLER, Markus**
  **1620 Nyon (CH)**

(74) Vertreter: **Vossius & Partner Patentanwälte Rechtsanwälte mbB Siebertstrasse 3 81675 München (DE)**

(56) Entgegenhaltungen:
**EP-A2- 1 944 059      DE-A1-102009 025 407**
**DE-A1-102012 002 437**

**Beschreibung**

Die Erfindung betrifft eine Vorrichtung zur effektiven invasiven Mehrsegment-Neurostimulation.

Bei Patienten mit neurologischen oder psychiatrischen Erkrankungen, z. B. Morbus Parkinson, essentiellem Tremor, Epilepsie, Funktionsstörungen nach Schlaganfall, Dystonie oder Zwangserkrankungen, sind Nervenzellverbände in umschriebenen Bereichen des Gehirns, z. B. des Thalamus und der Basalganglien krankhaft, z. B. übersteigert synchron, aktiv. In diesem Fall bildet eine große Anzahl von Neuronen synchron Aktionspotentiale aus, d. h., die beteiligten Neuronen feuern übermäßig synchron. Beim Gesunden hingegen feuern die Neuronen in diesen Hirngebieten qualitativ anders, z. B. auf unkorrelierte Weise.

Beim Morbus Parkinson verändert die pathologisch synchrone Aktivität die neuronale Aktivität in anderen Hirngebieten, z. B. in Arealen der Großhirnrinde wie dem primär motorischen Cortex. Dabei zwingt die pathologisch synchrone Aktivität im Bereich des Thalamus und der Basalganglien beispielsweise den Großhirnrindenarealen ihren Rhythmus auf, so dass schließlich die von diesen Arealen gesteuerten Muskeln pathologische Aktivität, z. B. ein rhythmisches Zittern (Tremor), entfalten.

Zur Behandlung von medikamentös nicht hinreichend behandelbaren Parkinsonpatienten wird die tiefe Hirnstimulation eingesetzt. Hierbei werden Tiefenelektroden in speziellen Hirngebieten, z. B. im Nucleus subthalamicus, implantiert. Zur Linderung der Symptome wird über die Tiefenelektroden eine elektrische Reizung durchgeführt. Bei der Standard-Hochfrequenz-Stimulation zur Behandlung der Parkinsonschen Erkrankung wird eine sogenannte Hochfrequenz-Dauerreizung bei Frequenzen von über 100 Hz durchgeführt. Diese Behandlungsart hat keine lang anhaltenden therapeutischen Effekte (vgl. P. Temperli, J. Ghika, J.-G. Villemure, P. Burkhard, J. Bogousslavsky, and F. Vingerhoets: How do parkinsonian signs return after discontinuation of subthalamic DBS? Neurology 60, 78 (2003)). Mit weniger Reizung, z. B. Reizstrom, kommt die "Coordinated Reset"-Stimulation (CR-Stimulation) aus, die obendrein lang anhaltende therapeutische Effekte haben kann (vgl. P. A. Tass, L. Qin, C. Hauptmann, S. Doveros, E. Bezard, T. Boraud, W. G. Meissner: Coordinated reset neuromodulation has sustained after-effects in parkinsonian monkeys. Annals of Neurology 72, 816-820 (2012); I. Adamchic, C. Hauptmann, U. B. Barnikol, N. Pawelcyk, O.V. Popovych, T. Barnikol, A. Silchenko, J. Volkmann, G. Deuschl, W. Meissner, M. Maarouf, V. Sturm, H.-J. Freund, P. A. Tass: Coordinated Reset Has Lasting Aftereffects in Patients with Parkinson's Disease. Movement Disorders 29, 1679 (2014)).

Vorrichtungen zur Stimulation von Neuronen mittels implantierter Stimulationseinheiten sind u.a. aus den folgenden Dokumenten bekannt: DE 10 2009 025407 A1, DE 10 2012 002437 A1 und EP 1 944 059 A2.

Bei anderen Erkrankungen, z. B. bei medikamentös nicht ausreichend behandelbaren Epilepsien, werden neben Tiefenelektroden auch andere, z. B. epikortikale oder epidurale Elektroden, implantiert. Bei weiteren Erkrankungen, z. B. chronischen Schmerz-Syndromen, ist es üblich, nicht nur mittels Tiefenelektroden im Gehirn, sondern auch mittels z. B. epiduralen Elektroden das Rückenmark zu reizen. Anders als die CR-Stimulation haben die meisten anderen Stimulationsarten keine lang anhaltenden therapeutischen Effekte.

Therapeutische Effekte können auch durch direkte Stimulation des Hirngewebes bzw. Rückenmarks mit Licht, z. B. über implantierte Lichtleiter, erzielt werden. Hierbei können auch unterschiedliche raum-zeitliche Stimulationsmuster, wie z. B. die CR-Stimulation, zur Anwendung kommen.

Um Nebenwirkungen der elektrischen Reizung zu vermindern, welche insbesondere durch anatomische Inhomogenitäten hervorgerufen sind, werden Mehr- bzw. Vielkanalelektroden verwandt (vgl. H. C. Martens, E. Toader, M. M. Decre, et al.: Spatial steering of deep brain stimulation volumes using a novel lead design. Clinical neurophysiology 122, 558-566 (2011); J. Buhlmann, L. Hofmann, P. A. Tass, C. Hauptmann: Modeling of a segmented electrode for desynchronizing deep brain stimulation. Frontiers in Neuroengineering 4, 15 (2011)). Derartige Mehr- bzw. Vielkanalelektroden zielen darauf ab, z. B. bei ungünstig platzierten Tiefenelektroden die traditionelle Hochfrequenz-Stimulation möglichst auf das Zielgebiet einzugrenzen, um benachbarte Strukturen nicht mit zu stimulieren.

Obgleich die tiefe Hirnstimulation mittels invasiver CR-Stimulation lang anhaltende therapeutische Effekt ermöglicht, hat dieser Ansatz Limitationen. Die CR-Stimulation kann Nebenwirkungen hervorrufen, z. B. durch die nicht gewünschte Reizung von benachbarten Strukturen infolge der Ausbreitung von Reizen, z. B. Reizströmen, in Bereiche außerhalb des Zielgebiets oder durch die aus anatomischen Gründen schwer vermeidbare gleichzeitige Reizung von Strukturen, z. B. von Faserbündeln, innerhalb des jeweiligen Zielgebiets (vgl. C. Moreau, L. Defebvre, A. Destee, et al.: STN-DBS frequency effects on freezing of gait in advanced Parkinson disease. Neurology 71, 80-84 (2008); M. Jahanshahi, I. Obeso, C. Baunez, et al.: Parkinson's disease, the subthalamic nucleus, inhibition, and impulsivity. Movement Disorders 30, 128-140 (2015)). Derartige Situationen ergeben sich z. B. durch die charakteristische enge anatomische Nachbarschaft von bei der Elektrodenimplantation anvisiertem Zielpunkt und anderen anatomischen Strukturen, deren Reizung zu Nebenwirkungen führt, durch spezielle individuelle anatomische Randbedingungen (z. B. im Sinne der Lage von Blutgefäßen, die bei der Implantation der Elektroden geschont werden müssen) oder auch durch suboptimale oder gar fehlerhafte Elektrodenimplantation.

Weiterhin tritt die therapeutische Wirkung bei manchen Patienten verzögert ein und/oder bildet sich nicht vollumfänglich aus. Es können räumlich ausgedehnte Synchronisationsprozesse an verschiedenen Stellen, z. B. durch unterschiedliche dynamische Charakteristika, insbesondere unterschiedliche dominante Frequenzen der oszillatorischen Aktivität, unterschiedlich auf dieselben Reize reagieren. Insbesondere wird die Effektivität der CR-Stimulation reduziert, wenn räumlich bzgl. ihrer dominanten Frequenz inhomogene Synchronisationsprozesse mit Reizen derselben Rythmizität, d. h. Stimulationsperiode, stimuliert werden. Eine räumlich inhomogene Verteilung der dominanten Frequenzen von neuronalen Synchronisationsprozessen kann z. B. aus der somatotopen Anordnung von Neuronen resultieren: Die Neuronen unterschiedlicher räumlicher Teilbereiche sind für unterschiedliche Körper- sowie Gliedmaßen-Teile zuständig. Unterschiedliche Abschnitte von Gliedmaßen, z. B. Hand vs. Oberarm, haben unterschiedliche mechanische Eigenfrequenzen, was im Krankheitsfall das Auftreten von unterschiedlichen dominanten Frequenzen befördert.

Den beiden vorstehend beschriebenen Limitationen liegt zugrunde, dass eine optimale Stimulation durch anatomische sowie funktionale Inhomogenitäten erschwert wird. Mit anatomischen Inhomogenitäten ist hier gemeint, dass der Anteil an Neuronen bestimmter Art und insbesondere an Fasern, welche durch das Zielgebiet laufen, typischerweise räumlich inhomogen ist. Funktionale Inhomogenitäten sind dadurch bedingt, dass bei räumlich ausgedehnten neuronalen Synchronisationsprozessen die charakteristischen dynamischen Parameter in verschiedenen Bereichen zum Teil stark variieren können. So können z. B. die dominanten Frequenzen, z. B. im Sinne einer spektralen Analyse, räumlich inhomogen verteilt sein und zudem zeitlich stark variieren.

Der Erfindung liegt die Aufgabe zugrunde, eine Vorrichtung sowie ein Verfahren zur Stimulation von Neuronen anzugeben, mit denen sich im Vergleich zum Stand der Technik die Reizung deutlich besser den lokalen anatomischen und funktionalen Gegebenheiten anpassen lässt. Hierdurch sollen Nebenwirkungen signifikant vermindert und die therapeutische Wirkung signifikant verbessert werden.

Die der Erfindung zugrunde liegende Aufgabenstellung wird durch die Merkmale der unabhängigen Ansprüche gelöst. Vorteilhafte Weiterbildungen und Ausgestaltungen der Erfindung sind in den Unteransprüchen angegeben.

Die Erfindung wird nachfolgend in beispielhafter Weise unter Bezugnahme auf die Zeichnungen näher erläutert. In diesen zeigen:

Fig. 1           eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer ersten Ausgestaltung;

Fig. 2           eine schematische Darstellung eines Zielareals mit räumlich voneinander getrennten neuronalen Synchronisationsprozessen;

Fig. 3           eine schematische Darstellung eines Zielareals mit einem räumlich umschriebenen Bereich, in dem sich unterschiedliche Teilbereiche eines neuronalen Synchronisationsprozessen befinden;

Fig. 4           eine schematische Darstellung einer Vielkanal-Elektrode zur Ableitung von Messsignalen und/oder direkten Stimulation des Zielareals;

Fig. 5           eine schematische Darstellung einer Vielkanal-Elektrode zur indirekten Stimulation des Zielareals;

Fig. 6           eine schematische Darstellung zweier Vielkanal-Elektroden zur Ableitung von Messsignalen und/oder direkten bzw. indirekten Stimulation des Zielareals;

Fig. 7           eine schematische Darstellung einer Vorrichtung zur Unterdrückung einer krankhaft synchronen und oszillatorischen neuronalen Aktivität und insbesondere zur Desynchronisierung von Neuronen mit einer krankhaft synchronen und oszillatorischen Aktivität gemäß einer zweiten Ausgestaltung;

Fig. 8           eine schematische Darstellung einer Vielkanal-Elektrode mit Gruppen und Untergruppen von Kontakten;

Fig. 9           schematische Darstellungen unterschiedlicher CR-Reizfolgen zur Stimulation von Neuronen;

Fig. 10         eine schematische Darstellung einer Mehrsegment-CR-Stimulation;

Fig. 11         eine schematische Darstellung einer iterativen Mehrkanal-Stimulation;

Fig. 12         eine schematische Darstellung des Prinzips der iterativen Mehrkanal-Stimulation;

Fig. 13         schematische Darstellungen der für die iterative Mehrkanal-Stimulation verwendeten Kontakte einer Vielkanal-Elektrode;

Fig. 14 und 15    schematische Darstellungen von Vorrichtungen zur invasiven elektrischen Stimulation von Neuronen; und

Fig. 16 und 17    schematische Darstellungen von Mehrkanal-Elektroden mit Gruppen und Untergruppen von Kontakten.

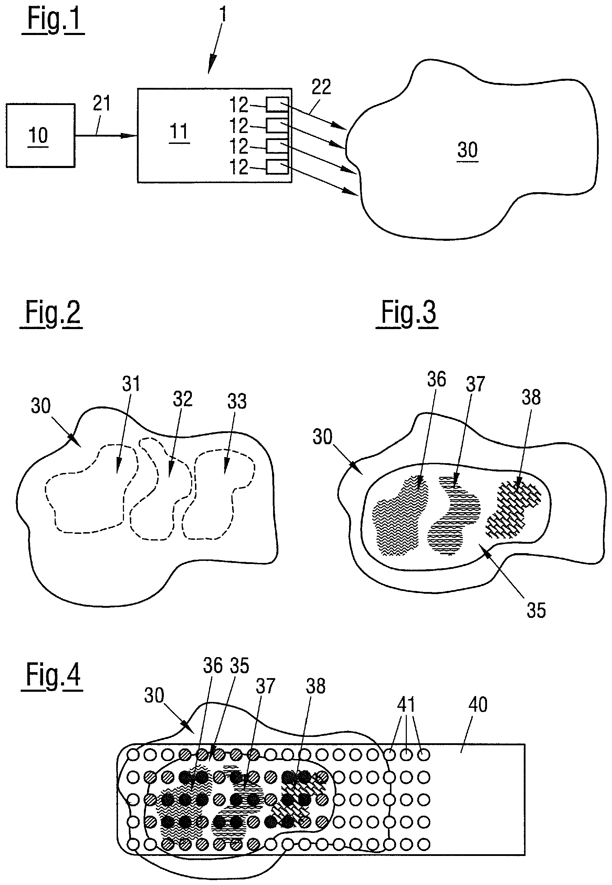

In Fig. 1 ist schematisch eine Vorrichtung 1 zur Stimulation von Neuronen mit einer krankhaft synchronen und oszillatorischen neuronalen Aktivität dargestellt. Die Vorrichtung 1 besteht aus einer Steuereinheit 10 und einer Stimulationseinheit 11, die eine Mehrzahl von Stimulationselementen 12 aufweist. In Fig. 1 sind beispielhaft vier Stimulationselemente 12 dargestellt. Die Stimulationseinheit 11 kann selbstverständlich aber auch eine andere Zahl von Stimulationselementen 12 aufweisen. Im Fall von elektrischer Stimulation kann es sich bei den Stimulationselementen 12 z. B. um Stimulationskontaktflächen einer oder mehrerer Elektroden zur Applikation elektrischer Reize handeln. Falls optisch stimuliert wird, können z. B. Lichtleiter als Stimulationselemente 12 eingesetzt werden, um das neuronale Gewebe an den gewünschten Stellen mit Lichtreizen zu stimulieren.

Während des Betriebs der Vorrichtung 1 führt die Steuereinheit 10 eine Steuerung der Stimulationseinheit 11 durch. Dazu erzeugt die Steuereinheit 10 Steuersignale 21, die von der Stimulationseinheit 11 entgegengenommen werden.

Die Stimulationseinheit 11 wird operativ in den Körper des Patienten implantiert und erzeugt anhand der Steuersignale 21 Reize 22, insbesondere elektrische und/oder optische Reize 22, die einem Zielareal 30 im Gehirn und/oder Rückenmark des Patienten verabreicht werden. Die Reize 22 sind dazu ausgelegt, bei einer Verabreichung an den Patienten die krankhaft synchrone und oszillatorische neuronale Aktivität zu unterdrücken und insbesondere die Neuronen mit der krankhaft synchronen und oszillatorischen Aktivität zu desynchronisieren.

Die Steuereinheit 10 kann eine nicht-invasive Einheit sein, d. h., während des Betriebs der Vorrichtung 1 befindet sie sich außerhalb des Körpers des Patienten und wird nicht operativ in den Körper des Patienten implantiert.

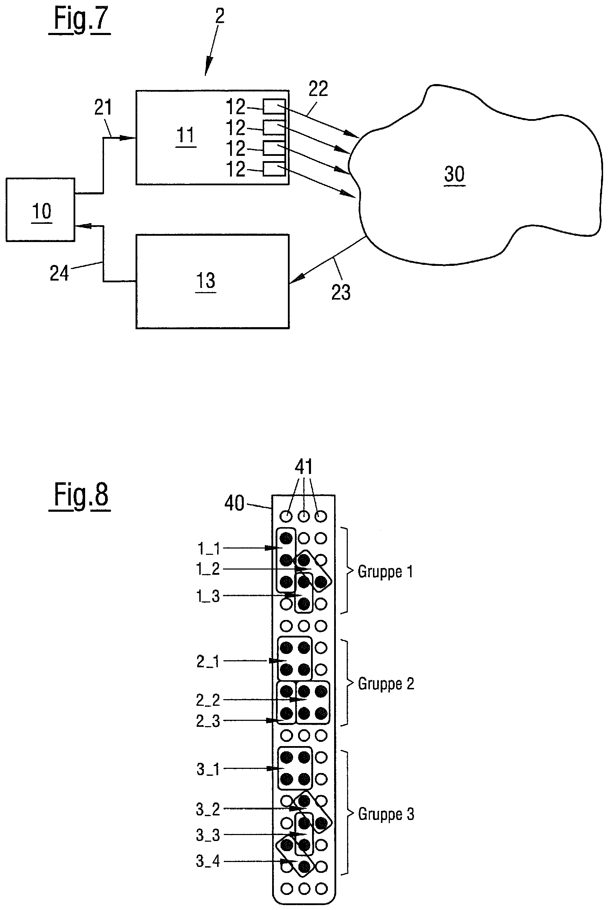

Die Vorrichtung 1 und die weiter unten, im Zusammenhang mit Fig. 7 beschriebene Vorrichtung 2 können insbesondere zur Behandlung von neurologischen oder psychiatrischen Erkrankungen eingesetzt werden, z. B. Morbus Parkinson, essentiellem Tremor, Tremor infolge von Multipler Sklerose sowie anderen pathologischen Tremores, Dystonie, Epilepsie, Depression, Bewegungsstörungen, Kleinhirnerkrankungen, Zwangserkrankungen, Demenzerkrankungen, Morbus Alzheimer, Tourette-Syndrom, Autismus, Funktionsstörungen nach Schlaganfall, Spastik, Tinnitus, Schlafstörungen, Schizophrenie, Reizdarm-Syndrom, Suchterkrankungen, Borderline-Persönlichkeitsstörung, Aufmerksamkeits-Defizit-Syndrom, Aufmerksamkeits-Defizit-Hyperaktivitäts-Syndrom, Spielsucht, Neurosen, Fresssucht, Magersucht, Essstörungen, Burnout-Syndrom, Fibromyalgie, Migräne, Cluster-Kopfschmerz, allgemeiner Kopfschmerz, Neuralgie, Ataxie, Tic-Störung oder Hypertonie, sowie weiteren Erkrankungen, die durch krankhaft gesteigerte neuronale Synchronisation gekennzeichnet sind.

Die vorstehend genannten Krankheiten können durch eine Störung der bioelektrischen Kommunikation von Neuronenverbänden, die in spezifischen Schaltkreisen zusammengeschlossen sind, verursacht werden. Hierbei generiert eine Neuronenpopulation anhaltend krankhafte neuronale Aktivität und möglicherweise eine damit verbundene krankhafte Konnektivität (Netzwerkstruktur). Dabei bildet eine große Anzahl von Neuronen synchron Aktionspotentiale aus, d. h., die beteiligten Neuronen feuern übermäßig synchron. Hinzu kommt, dass die kranke Neuronenpopulation eine oszillatorische neuronale Aktivität aufweist, d. h., die Neuronen feuern rhythmisch. Im Fall von neurologischen oder psychiatrischen Erkrankungen liegt die mittlere Frequenz der krankhaften rhythmischen Aktivität der betroffenen Neuronenverbände etwa im Bereich von 1 bis 30 Hz, kann aber auch außerhalb dieses Bereichs liegen. Bei gesunden Menschen feuern die Neuronen hingegen qualitativ anders, z. B. auf unkorrelierte Weise.

Räumliche ausgedehnte pathologische neuronale Synchronisationsprozesse können räumlich inhomogen sein und müssen insbesondere räumlich nicht zusammenhängend sein. Sie können aus räumlich getrennten, aber synaptisch miteinander verbundenen Teilbereichen bestehen. Dies ist beispielhaft in Fig. 2 gezeigt. Dort ist schematisch das Zielareal 30 dargestellt, in dem sich in unterschiedlichen Teilbereichen 31, 32 und 33 räumlich voneinander getrennte neuronale Synchronisationsprozesse befinden.

Pathologische neuronale Synchronisationsprozesse können auch räumlich umschrieben sein, aber dennoch durch funktionale Charakteristika räumlich inhomogen sein. Ein Beispiel für derartige Synchronisationsprozesse zeigt Fig. 3. Hier befinden sich in einem räumlich umschriebenen Bereich 35 unterschiedliche Teilbereiche 36, 37 und 38 des neuronalen Synchronisationsprozesses.

In den Teilbereichen 31 bis 33 bzw. 36 bis 38 können unterschiedliche dominante Frequenzen der pathologischen Oszillation der Neuronen vorherrschen. Die jeweiligen dominanten Frequenzen oder auch andere charakteristische funktionale Merkmale können durch Viel- bzw. Mehrkanal-Ableitungen erfasst werden. Hierbei werden über die jeweiligen Kontaktstellen z. B. lokale Feldpotentiale (LFP) abgeleitet. Es können auch Einzelzell-Ableitungen durchgeführt werden. Dem Fachmann ist bekannt, wie mit Standard-Datenanalyse-Methoden die zugrunde liegenden oszillatorischen neuronalen Aktivitäten abgeschätzt werden.

Fig. 4 zeigt schematisch eine Vielkanal-Elektrode 40, die als Stimulationseinheit 11 dient und eine Vielzahl von in einem Array angeordneten elektrisch leitfähigen Kontakten bzw. Stimulationskontaktflächen 41 aufweist, welche die Stimulationselemente 12 darstellen. In der vorliegenden Ausführungsform sind die Kontakte 41 einzeln ansteuerbar, so dass über jeden Kontakt 41 ein gewünschter elektrischer Reiz 22 appliziert werden kann. Ferner können die Kontakte 41 auch, wie weiter unten noch detaillierter beschrieben wird, zur Messung von neuronaler Aktivität eingesetzt werden.

In Fig. 4 ist beispielhaft eine elektrische Stimulation der Teilbereiche 36 bis 38 des Zielareals 30 dargestellt, in denen unterschiedliche dominante Frequenzen der pathologischen neuronalen Oszillation vorherrschen. Das räumliche Profil der Amplitude der über die jeweiligen Kontakte 41 gemessenen Signale bzw. der spektralen Power ist schematisch durch eine unterschiedliche Füllung der Kontakte 41 dargestellt. Je dunkler die Füllung eines Kontakts 41 ist, desto größer ist die im neuronalen Gewebe an der betreffenden Stelle gemessene dominante Frequenz der pathologischen synchronen Oszillation.

Bei der direkten Stimulation sind die Kontakte 41 unmittelbar auf dem zu stimulierenden Bereich 35 platziert. Hierbei können die Somata, Axone und Dendriten der entsprechenden Neuronenpopulationen direkt gereizt werden. In dem vorliegenden Beispiel werden die Teilbereiche 36 bis 38 über die den jeweiligen Teilbereichen 36 bis 38 zugeordneten Kontakte 41 mit der dunklen Füllung stimuliert. Jedem der Teilbereiche 36 bis 38 ist dabei eine Gruppe von Kontakten 41 zugeordnet.

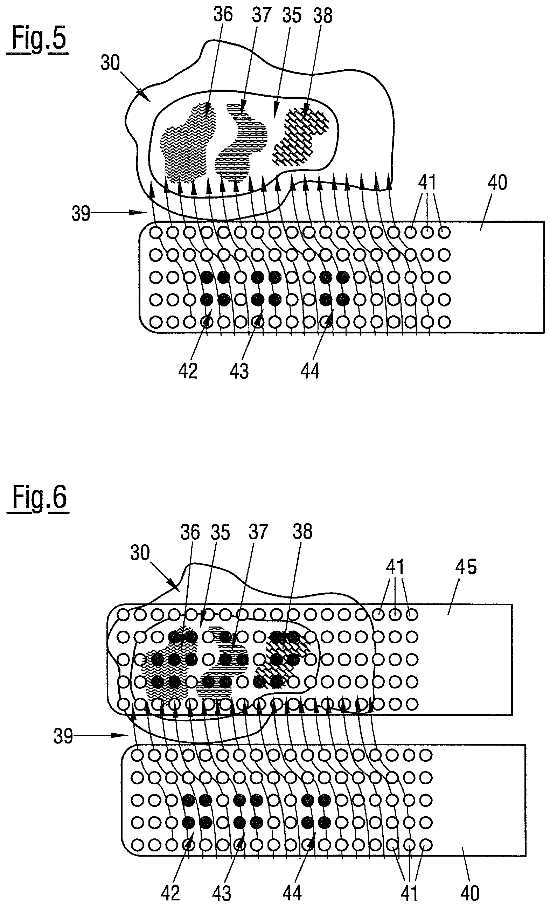

Räumlich separate bzw. durch funktionale Charakteristika getrennte Teilbereiche können über unterschiedliche Kontakt-Gruppen auch indirekt stimuliert werden, wie dies beispielhaft in Fig. 5 gezeigt ist. Die Vielkanal-Elektrode 40 ist hier nicht direkt auf den Teilbereichen 36 bis 38 platziert, vielmehr werden Fasern 39, welche zu den entsprechenden Teilbereichen 36 bis 38 führen und/oder aus ihnen entspringen, gereizt. In dem in Fig. 5 dargestellten Ausführungsbeispiel sind Gruppen 42, 43 und 44 aus jeweils mehreren Kontakten 41 gebildet und mit den Gruppen 42, 43 und 44 werden die afferenten Fasern 39, die zu den Teilbereichen 36, 37 bzw. 38 führen, stimuliert. Die Kontakte 41 der Gruppen 42 bis 44 sind in Fig. 5 mit einer dunklen Füllung dargestellt.

Es können auch Kombinationen von direkter und indirekter Reizung durchgeführt werden. Beispielhaft ist eine derartige Kombination in Fig. 6 dargestellt. Hier ist zusätzlich zu der indirekt stimulierenden Vielkanal-Elektrode 40 aus Fig. 5 eine weitere Vielkanal-Elektrode 45 direkt über dem Bereich 35 platziert. Bei einer kombinierten direkten und indirekten Stimulation können manche der Teilbereiche 36 bis 38 ausschließlich direkt, die anderen ausschließlich indirekt stimuliert werden. Es können z. B. die Teilbereiche 36 und 38 über die Kontakte 41 der Vielkanal-Elektrode 45 gereizt werden, der Teilbereich 37 hingegen über die Kontakte 41 der Gruppe 43 der Vielkanal-Elektrode 40. Prinzipiell kann auch eine gleichzeitige und/oder zeitlich alternierende kombinierte direkte und indirekte Reizung desselben Teilbereichs erfolgen.

Bei der direkten und/oder indirekten elektrischen Stimulation können die dem Fachmann bekannten Formen der bipolaren Stimulation zwischen Paaren von Kontakten 41 als auch der unipolaren Stimulation zwischen Kontakten 41 und einer gemeinsamen Masse angewandt werden.

Die in Fig. 1 dargestellte Vorrichtung 1 zur Stimulation von Neuronen mit einer krankhaft synchronen und oszillatorischen neuronalen Aktivität führt eine sogenannte "open loop"-Stimulation durch, d. h., eine Stimulation ohne Sensoren, die zur Rückmeldung und/oder Steuerung der Stimulation verwendet werden.

Fig. 7 zeigt schematisch eine Vorrichtung 2 zur Stimulation von Neuronen mit einer krankhaft synchronen und oszillatorischen neuronalen Aktivität, mit der sich eine "closed loop"-Stimulation durchführen lässt. Die Vorrichtung 2 ist eine Weiterbildung der in Fig. 1 dargestellten Vorrichtung 1 und enthält genauso wie die Vorrichtung 1 eine Steuereinheit 10 und eine implantierbare Stimulationseinheit 11, welche die gleichen Funktionen und Eigenschaften wie die oben beschriebenen Steuer- und Stimulationseinheiten 10, 11 der Vorrichtung 1 aufweisen.

Darüber hinaus umfasst die Vorrichtung 2 eine Messeinheit 13. Die Messeinheit 13 nimmt ein oder mehrere am Patienten gemessene Messsignale 23 auf, wandelt diese gegebenenfalls in elektrische Signale 24 um und führt sie der Steuereinheit 10 zu. Insbesondere kann mittels der Messeinheit 13 die neuronale Aktivität in dem stimulierten Zielareal 30 oder einem mit dem Zielareal 30 verbundenen Gebiet gemessen werden, wobei die neuronale Aktivität dieses Gebiets mit der neuronalen Aktivität des Zielgebiets 30 hinreichend eng korreliert. Bei räumlich ausgedehnten Synchronisationsprozessen kann an verschiedenen Stellen des Zielareals 30 insbesondere die dominante Frequenz der oszillatorischen Aktivität mit Hilfe der Messeinheit 13 gemessen werden. Mittels der Messeinheit 13 kann auch eine nicht-neuronale, z. B. muskuläre Aktivität oder die Aktivierung des autonomen Nervensystems, gemessen werden, sofern diese mit der neuronalen Aktivität des Zielgebiets hinreichend eng korreliert sind. Weiterhin kann der durch die Reize 22 erzielte Stimulationseffekt mit Hilfe der Messeinheit 13 überwacht werden.

Die Messeinheit 13 enthält einen oder mehrere Sensoren, die es insbesondere ermöglichen, die Amplitude der pathologischen oszillatorischen Aktivität aufzunehmen.

Die Sensoren können in den Körper des Patienten implantiert sein. Als invasive Sensoren können beispielsweise epikortikale Elektroden, Tiefenhirnelektroden zur Messung von z. B. lokalen Feldpotentialen, sub- oder epidurale Hirnelektroden, subkutane EEG-Elektroden und sub- oder epidurale Rückenmarkselektroden dienen. Die Tiefenelektroden zur Messung der lokalen Feldpotentiale können auch baulich vereint oder sogar identisch mit den für die Stimulation verwendeten Viel- bzw. Mehrkanal-Elektroden sein. Die Kontakte der Viel- bzw. Mehrkanal-Elektroden können derart platziert werden, dass sie relevante neuronale Feedback-Signale ableiten können. Bei der in Fig. 6 dargestellten Ausführungsform kann beispielsweise über die Vielkanal-Elektrode 45 die neuronale Aktivität abgeleitet werden, während über dieselbe Vielkanal-Elektrode 45 direkt und/oder über die Vielkanal-Elektrode 40 indirekt stimuliert wird.

Alternativ können nicht-invasive Sensoren eingesetzt werden, z. B. chronisch oder intermittent genutzte Elektroenzephalographie (EEG)- oder Elektromyographie (EMG)-Elektroden oder Magnetenzephalographie (MEG)-Sensoren. Die neuronale Aktivität kann auch durch Detektion charakteristischer Bewegungsmuster wie Tremor, Akinese oder epileptische Anfälle mit Hilfe eines Akzelerometers oder Gyroskops oder indirekt durch Messung der Aktivierung des autonomen Nervensystems mittels Messung des Hautleitwiderstands ermittelt werden. Es können auch Befindlichkeitswerte, die vom Patienten in portable Geräte, z. B. Smartphones, eingegeben werden, zur Kontrolle des Stimulationserfolgs verwandt werden. Derartige Befindlichkeitswerte können auch über Kurzfragebögen ermittelt werden.

Die Steuereinheit 10 verarbeitet die Signale 24, z. B. können die Signale 24 verstärkt und/oder gefiltert werden, und analysiert die verarbeiteten Signale 24. Die Steuereinheit 10 ermittelt insbesondere die dominante Frequenz der oszillatorischen Aktivität für die verschiedenen Teilbereiche des Zielareals 30 und überprüft anhand der in Reaktion auf die Applikation der Reize 22 aufgenommenen Messsignale 23 den Stimulationserfolg.

Die Stimulation des Zielareals 30 erfolgt insbesondere mittels einer CR-Stimulation. Im Gehirn und/oder Rückenmark des Patienten weist mindestens eine Neuronenpopulation eine wie vorstehend beschrieben krankhaft synchrone und oszillatorische neuronale Aktivität auf. Die Stimulationseinheit 11 bzw. die Vielkanal-Elektroden 40, 45 stimulieren die krankhaft aktive Neuronenpopulation im Gehirn und/oder Rückenmark mit den elektrischen und/oder optischen Reizen 22 entweder direkt oder indirekt. Die Reize 22 sind bei der CR-Stimulation so ausgestaltet, dass die zeitversetzte (oder phasenverschobene) Stimulation mit mindestens zwei Stimulationselementen 12 bzw. Kontakten 41 eine Desynchronisation der krankhaft synchronen Aktivität der Neuronenpopulation bewirkt. Eine durch die Stimulation bewirkte Senkung der Koinzidenzrate der Neuronen kann zu einer Senkung der synaptischen Gewichte und somit zu einem Verlernen der Tendenz zur Produktion krankhaft synchroner Aktivität führen.

Die bei der CR-Stimulation verabreichten Reize 22 bewirken in der Neuronenpopulation ein Zurücksetzen, einen sogenannten Reset, der Phase der neuronalen Aktivität der stimulierten Neuronen. Durch das Zurücksetzen wird die Phase der stimulierten Neuronen unabhängig von dem aktuellen Phasenwert auf einen oder nahe zu einem bestimmten Phasenwert, z. B. 0°, gesetzt (in der Praxis ist es nicht möglich, einen bestimmten Phasenwert exakt einzustellen, dies ist für eine erfolgreiche CR-Stimulation aber auch nicht erforderlich). Somit wird die Phase der neuronalen Aktivität der krankhaften Neuronenpopulation mittels einer gezielten Stimulation kontrolliert. Da die krankhafte Neuronenpopulation über die mehreren Stimulationselemente 12 bzw. Kontakte 41 an unterschiedlichen Stellen stimuliert wird, können die jeweiligen Phasen der neuronalen Aktivität von mehreren Subpopulationen der krankhaften Neuronenpopulation zu unterschiedlichen Zeitpunkten zurückgesetzt werden, indem die Reize 22 von den Stimulationselementen 12 bzw. Kontakten 41 zeitversetzt (oder phasenverschoben) appliziert werden. Im Ergebnis wird dadurch die krankhafte Neuronenpopulation, deren Neuronen zuvor synchron und mit gleicher Frequenz und Phase aktiv waren, in mehrere Subpopulationen mit unterschiedlichen Phasen aufgespalten. Innerhalb jeder der Subpopulationen sind die Neuronen nach dem Zurücksetzen der Phase weiterhin synchron und feuern auch weiterhin mit derselben pathologischen Frequenz, aber jede der Subpopulationen weist bezüglich ihrer neuronalen Aktivität die Phase auf, die ihr durch den von dem jeweiligen Stimulationselement 12 bzw. Kontakt 41 generierten Reiz 22 aufgezwungen wurde. Dies bedeutet, dass die neuronalen Aktivitäten der einzelnen Subpopulationen nach dem Zurücksetzen ihrer Phasen weiterhin einen in etwa sinusförmigen Verlauf mit derselben pathologischen Frequenz haben, aber unterschiedliche Phasen.

Wie vorstehend beschrieben stimulieren die Stimulationselemente 12 bzw. die Kontakte 41 mit den Reizen 22 unterschiedliche Subpopulationen. Es muss sich dabei aber nicht notwendigerweise um disjunkte, d. h. vollständig voneinander getrennte Subpopulationen handeln. Die von den Stimulationselementen 12 bzw. Kontakten 41 stimulierten Subpopulationen können einander auch überlappen.

Bedingt durch die krankhafte Interaktion zwischen den Neuronen ist der durch die Stimulation erzeugte Zustand mit mindestens zwei Subpopulationen instabil, und die gesamte Neuronenpopulation nähert sich schnell einem Zustand kompletter Desynchronisation, in welchem die Neuronen unkorreliert feuern. Der gewünschte Zustand, d. h. die komplette Desynchronisation, ist somit nach der zeitversetzten (oder phasenverschobenen) Applikation der phasenrücksetzenden Reize 22 nicht sofort vorhanden, sondern stellt sich meist innerhalb weniger Perioden oder gar in weniger als einer Periode der pathologischen Frequenz ein.

Eine Theorie zur Erklärung des Stimulationserfolgs basiert darauf, dass die letztlich gewünschte Desynchronisation durch die krankhaft gesteigerte Interaktion zwischen den Neuronen erst ermöglicht wird. Hierbei wird ein Selbstorganisationsprozess ausgenutzt, der für die krankhafte Synchronisation verantwortlich ist. Derselbe bewirkt, dass auf eine Aufteilung einer Gesamtpopulation in Subpopulationen mit unterschiedlichen Phasen eine Desynchronisation folgt. Im Gegensatz dazu würde ohne krankhaft gesteigerte Interaktion der Neuronen keine Desynchronisation erfolgen.

Darüber hinaus kann durch die CR-Stimulation eine Neuorganisation der Konnektivität der gestörten neuronalen Netzwerke erzielt werden, so dass lang anhaltende therapeutische Effekte bewirkt werden können. Der erzielte synaptische Umbau ist von großer Bedeutung für die wirksame Behandlung neurologischer oder psychiatrischer Erkrankungen.

Im Folgenden sollen die bei der Mehrsegment-Neurostimulation verabreichten Reizmuster näher beschrieben werden. In Analogie zu Fig. 4 ist in Fig. 8 beispielhaft eine Vielkanal-Elektrode 40 dargestellt, die eine Vielzahl von in einem Array angeordneten elektrisch leitfähigen Kontakten 41 aufweist, welche die Stimulationselemente 12 darstellen. In der vorliegenden Ausführungsform sind die Kontakte 41 einzeln ansteuerbar, so dass über jeden Kontakt 41 ein gewünschter elektrischer Reiz 22 appliziert werden kann. Ferner können die Kontakte 41 auch zur Ableitung von neuronaler Aktivität eingesetzt werden.

Beispielhaft sind in Fig. 8 drei Gruppen bzw. Segmente von Kontakten 41 dargestellt, die mit Gruppe 1, Gruppe 2 bzw. Gruppe 3 bezeichnet sind und jeweils mehrere Kontakte 41 umfassen. Die zu einer jeweiligen Gruppe gehörenden Kontakte 41 sind durch eine dunkle Füllung gekennzeichnet. Die Gruppen 1 bis 3 dienen dazu, unterschiedliche Teilbereiche eines Zielareals, z. B. die Teilbereiche 31 bis 33 aus Fig. 2 oder die Teilbereiche 36 bis 38 aus Fig. 3, mit übermäßig synchroner neuronaler Aktivität im Gehirn und/oder Rückenmark eines Patienten direkt und/oder indirekt zu stimulieren.

Für die Applikation einer CR-Stimulation besteht jede der Gruppen 1 bis 3 aus mehreren Untergruppen, wobei eine jeweilige Untergruppe einen oder mehrere Kontakte 41 umfassen kann. Die zu einer jeweiligen Untergruppe gehörenden Kontakte 41 sind in Fig. 8 durch einen Rahmen gekennzeichnet. Beispielhaft besteht in Fig. 8 die Gruppe 1 aus den Untergruppen 1_1, 1_2 und 1_3, die Gruppe 2 besteht aus den Untergruppen 2_1, 2_2 sowie 2_3 und die Gruppe 3 besteht aus den Untergruppen 3_1, 3_2, 3_3 und 3_4. Über die Untergruppen werden die einzelnen im Rahmen einer CR-Sequenz applizierten Reize 22 verabreicht. Die Kontakte 41 oder allgemein die Stimulationselemente 12 einer jeweiligen Untergruppe erzeugen stets zeitgleich den gleichen Reiz 22.

Die Stimulation über die unterschiedlichen Untergruppen kann über jeweils unabhängige Stromquellen erfolgen. Beispielsweise kann die Vorrichtung 1 über eine Anzahl von Stromquellen verfügen, die mindestens so groß ist wie die Anzahl der Untergruppen, über welche die Stimulation erfolgt.

Die Gruppen können durch Messungen und/oder Testreizungen identifiziert werden. Die Auswahl der Untergruppen innerhalb der jeweiligen Gruppen kann basierend auf folgenden Kriterien erfolgen: (i) Maximierung der wechselseitigen Entfernungen zwischen den jeweiligen bzw. benachbarten Untergruppen, (ii) Minimierung der wechselseitigen Kontaktzonen zwischen den jeweiligen bzw. benachbarten Untergruppen, (iii) vorbekannte anatomische und/oder physiologische Randbedingungen, (iv) Charakteristika von Reizantworten bei Reizung über verschiedene Untergruppen, (v) optimale Abdeckung der Bereiche mit stark ausgeprägter Synchronisation, d. h. z. B. großer LFP-Amplitude, gemäß Messung (wie in Fig. 4 illustriert) und (vi) minimaler räumlicher Überlapp des durch die unterschiedlichen Untergruppen jeweils stimulierten Gewebes.

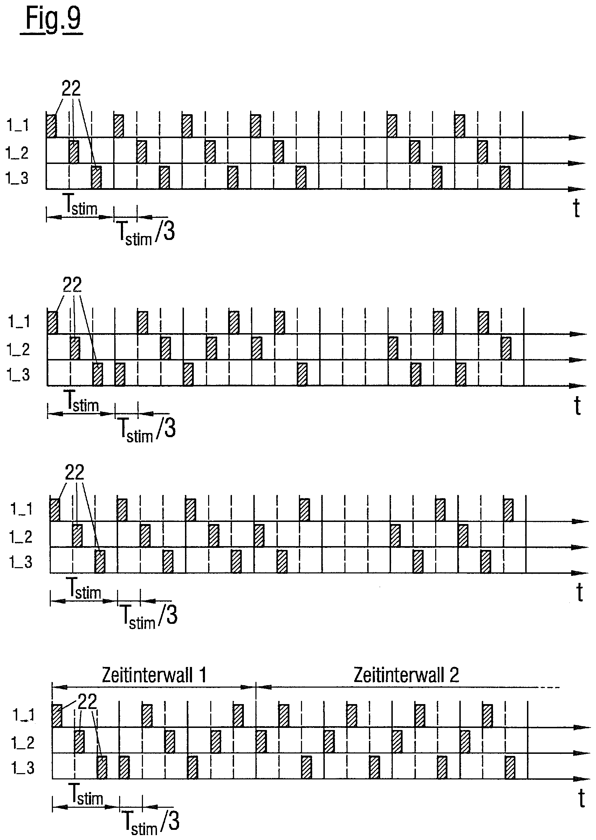

Über jede der Gruppen 1 bis 3 wird ein jeweiliger Teilbereich des Zielareals stimuliert. In Fig. 9 sind anhand der Gruppe 1 und deren Untergruppen 1_1, 1_2 und 1_3 beispielhaft vier verschiedene CR-Reizfolgen gezeigt, mit denen ein Teilbereich des Zielareals stimuliert werden kann.

In jeder der vier Teilabbildungen von Fig. 9 sind untereinander die von den Kontakten 41 der Untergruppen 1_1, 1_2 und 1_3 erzeugten Reize 22 gegen die Zeit t aufgetragen. Die Reize 22 werden in einem vorgegebenen Zeitraster, das aus aufeinanderfolgenden Zyklen besteht, erzeugt. Die einzelnen Zyklen sind in Fig. 9 durch durchgezogene, vertikale Linien voneinander abgegrenzt. Jeder Zyklus weist die Länge $T_{stim}$ auf. In jedem Zyklus, in dem eine Stimulation erfolgt, erzeugen die Untergruppen 1_1, 1_2 und 1_3 zusammen genau eine Sequenz von Reizen 22 und jede der Untergruppen 1_1, 1_2 und 1_3 erzeugt pro Sequenz genau einen Reiz 22, d. h., jede Sequenz besteht in dem vorliegenden Beispiel aus einer Abfolge von drei zeitversetzten Reizen 22, die insbesondere von jeweils unterschiedlichen Untergruppen 1_1, 1_2 und 1_3 generiert werden, wobei sich der Zeitversatz insbesondere auf die Anfangszeitpunkte der Reize 22 beziehen kann. Jeder Kontakt 41 einer jeweiligen Untergruppe 1_1, 1_2 und 1_3 erzeugt dabei den gleichen Reiz 22.

Jede Gruppe i kann grundsätzlich eine beliebige Anzahl $L_i$ von Untergruppen enthalten ($L_i \geq 2$), jedoch müssen bei einer Stimulation nicht zwingend alle $L_i$ Untergruppen verwendet werden, es kann beispielsweise auch nur eine Auswahl von $P_i$ der $L_i$ Untergruppen die Reize 22 erzeugen ($2 \leq P_i \leq L_i$), wobei innerhalb einer gegebenen Sequenz dann alle $P_i$ ausgewählten Untergruppen jeweils genau einen Reiz 22 erzeugen. Beispielsweise können von Zyklus zu Zyklus (oder in anderen Abständen) die zur Stimulation herangezogenen $P_i$ Untergruppen variiert werden, z. B. können pro Zyklus je drei verschiedene Untergruppen ausgewählt werden. Ferner kann auch die Anzahl $P_i$ der Untergruppen von Zyklus zu Zyklus (oder in anderen Abständen) variiert werden, z. B. kann mittels drei, vier oder fünf verschiedene Untergruppen in einem jeweiligen Zyklus stimuliert werden.

Bei $P_i$ Untergruppen der Gruppe i ergeben sich $P_i!$ mögliche unterschiedliche Sequenzen, wobei bei jeder dieser Sequenzen jede der $P_i$ Untergruppen genau einen Reiz 22 erzeugt. Es ist denkbar, alle $P_i!$ möglichen Sequenzen für die Stimulation heranzuziehen oder aus der Menge der $P_i!$ möglichen Sequenzen eine Untermenge für die Stimulation auszuwählen. Diese Untermenge kann auch in der Zeit gemäß stochastisch oder deterministisch oder gemischt stochastisch-deterministisch Regeln variieren. Die Abfolge der Sequenzen kann zufällig sein oder vor Beginn oder auch während der Stimulation festgelegt werden.

In der ersten, d. h. der obersten Teilabbildung von Fig. 9 ist die Reihenfolge, in welcher die Untergruppen 1_1, 1_2 und 1_3 die Reize 22 innerhalb eines Zyklus erzeugen, konstant. Ferner kann nach einer bestimmten Anzahl von Zyklen eine Pause eingehalten werden, in der keine Reize 22 appliziert werden. Die Dauer der Pause kann insbesondere $T_{stim}$ oder ein ganzzahliges Vielfaches von $T_{stim}$ betragen. Danach kann die Stimulation in der gleichen Weise wie vor der Pause fortgesetzt werden.

Die Gruppe 1 stimuliert einen bestimmten Teilbereich des Zielareals und jede der drei Untergruppen 1_1, 1_2 und 1_3 der Gruppe 1 stimuliert eine jeweilige Subpopulation dieses Teilbereichs. Während der Zyklen, in denen die Reize 22 appliziert werden, wird von jeder der Untergruppen 1_1, 1_2 und 1_3 der Reiz 22 periodisch mit der Periode $T_{stim}$ appliziert. Die Reize 22 bewirken eine Phasenrücksetzung der neuronalen Aktivität der jeweils stimulierten Subpopulation. Ferner beträgt die zeitliche Verzögerung zwischen innerhalb einer Sequenz zeitlich direkt aufeinanderfolgenden, von unterschiedlichen Untergruppen erzeugten Reizen 22 $T_{stim}/3$, da in dem vorliegenden Ausführungsbeispiel drei Untergruppen 1_1, 1_2 und 1_3 für die CR-Stimulation eingesetzt werden. Für den allgemeinen Fall von N für die Stimulation verwendeten Untergruppen würde die zeitliche Verzögerung zwischen innerhalb einer Sequenz zeitlich direkt aufeinanderfolgenden, von unterschiedlichen Untergruppen erzeugten Reizen 22 $T_{stim}/N$ betragen (von diesem Wert kann auch um z. B. bis zu $\pm$ 5% oder $\pm$ 10% oder eventuell um einen noch größeren Prozentsatz abgewichen werden). Die zeitliche Verzögerung $T_{stim}/N$ kann sich auf die Anfangszeitpunkte der Reize 22 beziehen. Die von unterschiedlichen Untergruppen erzeugten Reize 22 können bis auf die unterschiedlichen Startzeitpunkte identisch sein.

Die Periode $T_{stim}$, die zum einen die Dauer eines Zyklus und zum anderen die Periode angibt, mit der gleich bleibende Sequenzen sowie die von einer jeweiligen Untergruppe generierten Reize 22 wiederholt werden, kann nahe bei der mittleren Periode der dominanten pathologischen Oszillation (d. h. dem Inversen der dominanten Frequenz) der Neuronen im von der Gruppe 1 stimulierten Teilbereich des Zielareals mit der krankhaft synchronen und oszillatorischen neuronalen Aktivität liegen bzw. um bis zu $\pm$ 5% oder $\pm$ 10% von der mittleren Periode abweichen. Typischerweise liegt die Frequenz $f_{stim} = 1/T_{stim}$ im Bereich von 1 bis 30 Hz. Die dominante Frequenz der pathologischen Oszillation der zu stimulierenden Neuronen kann mit Hilfe der Messeinheit 13 gemessen werden. Es ist aber auch möglich, für die Periode der pathologischen Oszillation Literatur- oder Erfahrungswerte, die sich auf die jeweilige, zu behandelnde Krankheit beziehen, zu verwenden. Eine genauere Abschätzung der optimalen Frequenz $f_{stim} = 1/T_{stim}$ kann durch eine Analyse in einem gleitenden Zeitfenster mit dem Fachmann bekannten Datenanalyse-Verfahren durchgeführt werden. Z. B. kann in einem gleitenden Zeitfenster das absolute Maximum der spektralen Leistungsdichte in einem (medizinisch begründet) vordefinierten Frequenzintervall bestimmt werden. Anstatt der Bandpassfilterung können auch andere Datenvorverarbeitungsschritte verwendet werden, z. B. Wavelet-Analyse oder empirical mode decomposition (EMD). Es kann auch - gerade bei zeitweise und/oder infolge suboptimaler Lage der Sensoren verrauschten Signalen - eine Autokorrelationsfunktion berechnet werden.

Die phasenrücksetzenden Reize 22 können in allen vier Teilabbildungen von Fig. 9 beispielsweise Einzelreize oder auch zusammengesetzte Reize sein. Beispielsweise kann jeder Reiz 22 aus einem Pulszug mit 2 bis 100, insbesondere 2 bis 10 Einzelpulsen bestehen. Innerhalb eines Pulszugs werden die Einzelpulse ohne Unterbrechung mit einer sogenannten intra burst-Frequenz im Bereich von 50 bis 500 Hz, insbesondere im Bereich von 100 bis 200 Hz, wiederholt. Die intra burst-Frequenz innerhalb eines Pulszugs kann fest sein. Ferner können die Pulse eines Pulszugs identisch sein.

Während in der ersten Teilabbildung von Fig. 9 die Sequenzen konstant sind, ist in der zweiten Teilabbildung von Fig. 9 eine Ausgestaltung gezeigt, die eine Weiterbildung der in der ersten Teilabbildung gezeigten CR-Stimulation darstellt und bei der zu Beginn jedes Zyklus die Reihenfolge, in welcher die Untergruppen 1_1, 1_2 und 1_3 die phasenrücksetzenden Reize 22 erzeugen, variiert, insbesondere zufällig variiert wird. Beispielsweise erzeugen die Untergruppen 1_1, 1_2 und 1_3 in dem ersten in der zweiten Teilabbildung von Fig. 9 gezeigten Zyklus die Reize 22 in der Reihenfolge 1_1-1_2-1_3. Im zweiten Zyklus lautet die Reihenfolge 1_3-1_1-1_2 und im dritten Zyklus lautet die Reihenfolge 1_3-1_2-1_1.

Die dritte Teilabbildung von Fig. 9 zeigt eine Weiterentwicklung der in der zweiten Teilabbildung dargestellten CR-Stimulation. Der wesentliche Unterschied zur Stimulation gemäß der zweiten Teilabbildung besteht darin, dass bei der in der dritten Teilabbildung dargestellten CR-Stimulation die Sequenzen nur sehr langsam variiert werden. Insbesondere ist vorgesehen, dass die Reihenfolge, in welcher die Untergruppen 1_1, 1_2 und 1_3 innerhalb einer Sequenz die phasenrücksetzenden Reize 22 erzeugen, für mindestens 20 nacheinander generierte Sequenzen konstant gehalten und erst danach variiert wird. Eine CR-Stimulation mit derart langsam variierenden Sequenzen ist gegenüber der in der zweiten Teilabbildung von Fig. 9 gezeigten CR-Stimulation erheblich überlegen, da ihr gewünschter, d. h. therapeutischer Stimulationseffekt (i) stärker ausgeprägt ist, (ii) von Stimulationsepoche zu Stimulationsepoche deutlich weniger variiert und (iii) deutlich robuster gegenüber Schwankungen der Reizintensität, gegenüber Schwankungen charakteristischer Kenngrößen des Körpers bzw. Nervensystems sowie insbesondere gegenüber Variationen der Anfangswerte ist.

Wie oben beschrieben kann vorgesehen sein, dass die Sequenzen für mindestens 20 nacheinander generierte Sequenzen gleich bleiben und erst danach geändert werden. Es ist weiterhin denkbar, die Wiederholung derselben Sequenz zu erhöhen und die Reihenfolge, in welcher die Untergruppen 1_1, 1_2 und 1_3 pro Zyklus die Reize 22 erzeugen, für mindestens 25 oder mindestens 30 nacheinander generierte Sequenzen konstant zu halten. Es sei an dieser Stelle noch darauf hingewiesen, dass in der dritten Teilabbildung von Fig. 9 aus Gründen der Veranschaulichung die Sequenzen bereits nach weniger als 20 Sequenzen variiert werden. Dies ist jedoch lediglich als eine vereinfachte Darstellung einer im Vergleich zur zweiten Teilabbildung von Fig. 9 langsamen Sequenzvariation zu verstehen.

Gemäß einer Ausgestaltung wird bei der in der dritten Teilabbildung von Fig. 9 gezeigten CR-Stimulation nur die Reihenfolge, in welcher die Untergruppen 1_1, 1_2 und 1_3 pro Sequenz die Reize 22 erzeugen, variiert. Alle übrigen Stimulationsparameter können während der CR-Stimulation konstant bleiben.

Die Variation der Sequenzen kann z. B. stochastisch oder deterministisch oder gemischt stochastisch-deterministisch erfolgen.

Genauso wie in den ersten und zweiten Teilabbildungen können auch bei der CR-Stimulation gemäß der dritten Teilabbildung von Fig. 9 Zyklen vorgesehen sein, in denen Stimulationspausen eingehalten werden. So können während n aufeinanderfolgenden Zyklen Reize 22 erzeugt werden und während der darauffolgenden m Zyklen keine Reize 22 erzeugt werden, die dazu ausgelegt sind, die krankhaft synchrone und oszillatorische neuronale Aktivität zu unterdrücken, wobei n und m nicht-negative ganze Zahlen sind. Es ist jedoch denkbar, dass andere Reize, die nicht dazu ausgelegt sind, die krankhaft synchrone und oszillatorische neuronale Aktivität zu unterdrücken, während der Stimulationspausen insbesondere mit den Untergruppen 1_1, 1_2 und 1_3 appliziert werden. Weiterhin kann vorgesehen sein, dass die Untergruppen 1_1, 1_2 und 1_3 während der Stimulationspausen keinerlei Reize erzeugt. Das Muster aus n Zyklen mit Stimulation und m Zyklen ohne Stimulation kann periodisch fortgesetzt werden.

Sofern vorgesehen ist, die Sequenzen nach einer vorgegebenen Anzahl i von Sequenzen zu variieren ($i \geq 20$), werden gemäß einer Ausgestaltung die Zyklen ohne Stimulation nicht mitgezählt, d. h., es findet bei dieser Ausgestaltung eine Variation der Reihenfolge, in der die Untergruppen 1_1, 1_2 und 1_3 die Reize 22 generieren, erst dann statt, wenn tatsächlich in i Zyklen jeweils eine Sequenz von Reizen 22 appliziert wurde. Die Anzahl i, nach der jeweils die Sequenz variiert wird, kann z. B. gemäß stochastischen oder deterministischen oder gemischt stochastisch-deterministischen Regeln bestimmt werden.

Weiterhin kann die Variation der Sequenzen mit einem konstanten Rhythmus erfolgen, d. h., eine Variation findet beispielsweise stets nach i Zyklen statt.

Die CR-Stimulation mit langsam variierender Sequenz ist besonders geeignet, wenn mit überschwelligen Reizstärken stimuliert werden kann. Sie ist der CR-Stimulation mit fixer Sequenz oder der CR-Stimulation mit schnell variierender Sequenz dann typischerweise überlegen. Ist hingegen die Nebenwirkungsschwelle, also die zur Auslösung von Nebenwirkungen erforderliche Reizamplitude vermindert und/oder kommt es bei der Reizung zu Nebenwirkungen, so kann eine Zwei-Stufen-CR-Stimulation verwendet werden. Der Vorteil der Zwei-Stufen-CR-Stimulation ist, dass die erste Stufe mit unterschwelliger Reizstärke appliziert wird, während erst bei der zweiten Stufe überschwellig gereizt wird. Trotz der vergleichsweise besonders schwachen Reizstärke sind die therapeutischen Effekte gut und anhaltend.

Eine Zwei-Stufen-CR-Stimulation soll nachfolgend anhand der vierten, d. h. der untersten Teilabbildung von Fig. 9 beispielhaft erläutert werden. Bei der Zwei-Stufen-CR-Stimulation wird in der ersten Stufe mit rasch variierender Sequenz bei einer insbesondere geringen Reizstärke stimuliert und in der zweiten Stufe wird mit langsam variierender Sequenz bei einer insbesondere höheren Reizstärke stimuliert. Zur Realisierung der beiden Stimulationsstufen kann die Vielkanal-Elektrode 40 oder allgemein die Stimulationseinheit 11 in zwei verschiedenen Stimulationsmodi (oder Betriebsmodi) betrieben werden. Während eines ersten Zeitintervalls, welches in der vierten Teilabbildung von Fig. 9 als Zeitintervall 1 bezeichnet ist, betreibt die Steuereinheit 10 die Kontakte 41 der Gruppe 1 in einem ersten Stimulationsmodus. Im ersten Stimulationsmodus steuert die Steuereinheit 10 die Gruppe 1 derart an, dass die Untergruppen 1_1, 1_2 und 1_3 der Gruppe 1 Sequenzen von Reizen 22 repetitiv erzeugen und die Reihenfolge, in welcher die Untergruppen 1_1, 1_2 und 1_3 innerhalb einer Sequenz die phasenrücksetzenden Reize 22 erzeugen, für höchstens 5 nacheinander generierte Sequenzen konstant ist und danach variiert wird, wobei die Stärke der Reize 22 im ersten Stimulationsmodus insbesondere kleiner oder gleich einer vorgegebenen Reizstärke ist. Ansonsten können die Reize 22 genauso wie in den ersten bis dritten Teilabbildungen von Fig. 9 ausgestaltet sein. Das Muster, nach welchem die Reihenfolge, in welcher die Stimulationselemente innerhalb einer Sequenz die Reize erzeugen, für höchstens 5 nacheinander generierte Sequenzen konstant ist und danach variiert wird, kann mehrfach wiederholt werden.

Auf das erste Zeitintervall folgt ein zweites Zeitintervall, welches in der vierten Teilabbildung von Fig. 9 als Zeitintervall 2 bezeichnet ist. Insbesondere kann sich das zweite Zeitintervall unmittelbar dem ersten Zeitintervall, d. h. ohne eine dazwischen liegende Pause anschließen. Während des zweiten Zeitintervalls betreibt die Steuereinheit 10 die Untergruppen 1_1, 1_2 und 1_3 der Gruppe 1 in dem zweiten Stimulationsmodus. Im zweiten Stimulationsmodus steuert die Steuereinheit 10 die Untergruppen 1_1, 1_2 und 1_3 derart an, dass die Untergruppen 1_1, 1_2 und 1_3 Sequenzen von phasenrücksetzenden Reizen 22 repetitiv erzeugen und die Reihenfolge, in welcher die Untergruppen 1_1, 1_2 und 1_3 innerhalb einer Sequenz die Reize 22 erzeugen, für mindestens 25 nacheinander generierte Sequenzen konstant ist und danach variiert wird. Die Stärke der Reize 22 im zweiten Stimulationsmodus beträgt insbesondere mindestens das 1,3-fache der vorgegebenen Reizstärke. Das Muster, nach welchem die Reihenfolge, in welcher die Stimulationselemente innerhalb einer Sequenz die Reize erzeugen, für mindestens 25 nacheinander generierte Sequenzen konstant ist und danach variiert wird, kann mehrfach wiederholt werden.

Es kann für den Patienten vorteilhaft sein, den Wechsel vom ersten Stimulationsmodus in den zweiten Stimulationsmodus nicht abrupt, sondern fraktioniert durchzuführen. Ein abrupter Wechsel von einer unterschwelligen Stimulationsstärke im ersten Stimulationsmodus zu einer überschwelligen Stimulationsstärke im zweiten Stimulationsmodus kann sehr unangenehm, z. B. schmerzhaft sein. Um diesen Übergang angenehmer zu gestalten, kann man Gewöhnungseffekte ausnutzen, indem man im Rahmen des Übergangs vom ersten Zeitintervall zum zweiten Zeitintervall mehrmals zwischen den beiden Stimulationsmodi hin- und herschaltet. Das Ausmaß der Nebenwirkungen, z. B. Schmerzen, hängt nicht nur von der Stimulationsstärke, sondern auch von der Dauer der Reizapplikation ab. Durch Applikation kurzer Epochen im zweiten Stimulationsmodus kann das Einsetzen der Nebenwirkungen deutlich abgeschwächt werden. Es kann sogar zu Gewöhnungseffekten kommen, so dass die Nebenwirkungen im später dauerhaft applizierten zweiten Stimulationsmodus geringer ausfallen als ohne den fraktionierten Übergang. Die Dauer zwischen dem Hin- und Herschalten zwischen dem ersten und dem zweiten Stimulationsmodus kann im Rahmen des Übergangs zeitlich variieren, z. B. zunehmen.

Wie oben beschrieben ist in der ersten Stufe vorgesehen, dass die Sequenzen für maximal 5 nacheinander generierte Sequenzen gleich bleiben und danach geändert werden. Weiterhin kann die Variation der Sequenzen mit einem konstanten Rhythmus erfolgen, d. h., eine Variation findet beispielsweise stets nach $i_{Modus\_1}$ Zyklen statt, wobei $i_{Modus\_1}$ eine ganze Zahl von 1 bis 5 ist. Alternativ kann die Anzahl der Zyklen, nach der die Sequenz variiert wird, gemäß stochastischen oder deterministischen oder gemischt stochastisch-deterministischen Regeln bestimmt werden. In der vierten Teilabbildung von Fig. 9 findet aus Gründen der Veranschaulichung in jedem Zyklus des ersten Zeitintervalls eine Variation der Sequenz statt.

Gemäß einer Ausgestaltung wird bei der Zwei-Stufen-CR-Stimulation nur die Reihenfolge, in welcher die Untergruppen 1_1, 1_2 und 1_3 pro Sequenz die Reize 22 erzeugen, variiert. Alle übrigen Stimulationsparameter können während der CR-Stimulation konstant bleiben.

Die Variation der Sequenzen kann z. B. stochastisch oder deterministisch oder gemischt stochastisch-deterministisch erfolgen.

Es kann vorgesehen sein, dass die CR-Stimulation im ersten Stimulationsmodus kontinuierlich erfolgt, d. h., in aufeinander folgenden Zyklen werden stets Sequenzen von Reizen 22 erzeugt. Alternativ können aber auch Pausen während der CR-Stimulation, insbesondere während ganzer Zyklen, eingehalten werden. So können während $n_{Modus\_1}$ aufeinanderfolgenden Zyklen Reize 22 erzeugt werden und während der darauffolgenden $m_{Modus\_1}$ Zyklen keine Reize 22 erzeugt werden, die dazu ausgelegt sind, die krankhaft synchrone und oszillatorische neuronale Aktivität zu desynchronisieren, wobei $n_{Modus\_1}$ und $m_{Modus\_1}$ nicht-negative ganze Zahlen sind. Das Muster aus $n_{Modus\_1}$ Zyklen mit Stimulation und $m_{Modus\_1}$ Zyklen ohne Stimulation kann periodisch fortgesetzt werden.

Es ist denkbar, dass andere Reize, die nicht dazu ausgelegt sind, die krankhaft synchrone und oszillatorische neuronale Aktivität zu unterdrücken, während der Stimulationspausen insbesondere mit der Vielkanal-Elektrode 40 bzw. der Stimulationseinheit 11 appliziert werden. Alternativ erzeugt die Vielkanal-Elektrode 40 bzw. die Stimulationseinheit 11 während der Stimulationspausen keinerlei Reize.

Sofern vorgesehen ist, die Sequenzen nach einer vorgegebenen Anzahl $i_{Modus\_1}$ von Sequenzen zu variieren ($i_{Modus\_1} \leq 5$), werden gemäß einer Ausgestaltung die Zyklen ohne Stimulation nicht mitgezählt, d. h., es findet bei dieser Ausgestaltung eine Variation der Reihenfolge, in der die Untergruppen 1_1, 1_2 und 1_3 die Reize 22 generieren, erst dann statt, wenn tatsächlich in $i_{Modus\_1}$ Zyklen jeweils eine Sequenz von Reizen 22 appliziert wurde.

Die Stärke der Reize 22, d. h. die Amplitude der Reize 22, ist im ersten Stimulationsmodus kleiner oder gleich einer vorgegebenen Reizstärke. Die vorgegebene Reizstärke kann insbesondere unterschwellig in dem Sinne sein, dass die Reize 22 nur während der Stimulation desynchronisierende Effekte haben, die das Ende der Stimulation jedoch nicht überdauern, d. h., nach dem Ende der Stimulation mit den Reizen 22, deren Reizstärke die vorgegebene Reizstärke nicht übersteigt, verschwindet der desynchronisierende Effekt.

Durch die Stimulation im ersten Stimulationsmodus wird die Neuronenpopulation in dem von der Gruppe 1 stimulierten Teilbereich in einen Zustand gebracht, in dem sie für die nachfolgende Stimulation im zweiten Stimulationsmodus mit langsam variierender Sequenz und höherer Reizstärke deutlich empfänglicher ist.

Die Stimulation im zweiten Stimulationsmodus kann bis auf die Anzahl der Zyklen, nach denen die Sequenz variiert wird, und die Reizstärke die gleichen Ausgestaltungen aufweisen, wie die oben erläuterte Stimulation im ersten Stimulationsmodus. Im Folgenden werden die Unterschiede der Stimulation im zweiten Stimulationsmodus gegenüber der Stimulation im ersten Stimulationsmodus erläutert.

Die vierte Teilabbildung von Fig. 9 zeigt im zweiten Zeitintervall eine CR-Stimulation, bei der die Untergruppen 1_1, 1_2 und 1_3 repetitiv langsam variierende Sequenzen von Reizen 22 im zweiten Stimulationsmodus erzeugen. Die Reihenfolge, in welcher die Untergruppen 1_1, 1_2 und 1_3 innerhalb einer Sequenz die Reize 22 erzeugen, wird für mindestens 25 nacheinander generierte Sequenzen konstant gehalten und erst danach variiert. Es ist weiterhin denkbar, die Wiederholung derselben Sequenz zu erhöhen und die Reihenfolge, in welcher die Untergruppen 1_1, 1_2 und 1_3 pro Zyklus die Reize 22 erzeugen, im zweiten Stimulationsmodus für beispielsweise mindestens 30 oder mindestens 35 nacheinander generierte Sequenzen konstant zu halten.

Die Variation der Sequenzen kann im zweiten Stimulationsmodus mit einem konstanten Rhythmus erfolgen, d. h., eine Variation findet beispielsweise stets nach $i_{Modus\_2}$ Zyklen statt, wobei $i_{Modus\_2} \geq 25$ gilt. Alternativ kann die Anzahl der Zyklen, nach der die Sequenz variiert wird, gemäß stochastischen oder deterministischen oder gemischt stochastisch-deterministischen Regeln bestimmt werden.

Wie bei der Stimulation im ersten Stimulationsmodus kann auch bei der Stimulation im zweiten Stimulationsmodus nur die Reihenfolge, in welcher die Untergruppen 1_1, 1_2 und 1_3 pro Sequenz die Reize 22 erzeugen, variiert werden. Alle übrigen Stimulationsparameter können während der Stimulation konstant bleiben.

Die Variation der Sequenzen kann z. B. stochastisch oder deterministisch oder gemischt stochastisch-deterministisch erfolgen.

Die CR-Stimulation kann im zweiten Stimulationsmodus kontinuierlich erfolgen, d. h., in aufeinanderfolgenden Zyklen werden stets Sequenzen von Reizen 22 erzeugt. Alternativ können aber auch Pausen während der CR-Stimulation, insbesondere während ganzer Zyklen, eingehalten werden. So können während $n_{Modus\_2}$ aufeinanderfolgenden Zyklen Reize 22 erzeugt werden und während der darauffolgenden $m_{Modus\_2}$ Zyklen keine Reize 22 erzeugt werden, die dazu ausgelegt sind, die krankhaft synchrone und oszillatorische neuronale Aktivität zu desynchronisieren, wobei $n_{Modus\_2}$ und $m_{Modus\_2}$ nicht-negative ganze Zahlen sind. Das Muster aus $n_{Modus\_2}$ Zyklen mit Stimulation und $m_{Modus\_2}$ Zyklen ohne Stimulation kann periodisch fortgesetzt werden. Die Werte für $n_{Modus\_2}$ und $m_{Modus\_2}$ des zweiten Stimulationsmodus können, müssen aber nicht identisch sein mit den Werten für $n_{Modus\_1}$ bzw. $m_{Modus\_1}$ des ersten Stimulationsmodus.

Es ist denkbar, dass andere Reize, die nicht dazu ausgelegt sind, die krankhaft synchrone und oszillatorische neuronale Aktivität zu unterdrücken, während der Stimulationspausen insbesondere mit den Untergruppen 1_1, 1_2 und 1_3 appliziert werden. Alternativ erzeugen die Untergruppen 1_1, 1_2 und 1_3 während der Stimulationspausen keinerlei Reize.

Sofern vorgesehen ist, die Sequenzen nach einer vorgegebenen Anzahl $i_{Modus\_2}$ von Sequenzen zu variieren ($i_{Modus\_2} \geq 25$), werden gemäß einer Ausgestaltung die Zyklen ohne Stimulation nicht mitgezählt, d. h., es findet bei dieser Ausgestaltung eine Variation der Reihenfolge, in der die Untergruppen 1_1, 1_2 und 1_3 die Reize 22 generieren, erst dann statt, wenn tatsächlich in $i_{Modus\_2}$ Zyklen jeweils eine Sequenz von Reizen 22 appliziert wurde.

Die Stärke der Reize 22, d. h. die Amplitude der Reize 22, beträgt im zweiten Stimulationsmodus mindestens das 1,3-fache der vorgegebenen Reizstärke. Die Stärke der Reize 22 kann insbesondere so groß sein, dass man einen ausgeprägten und anhaltenden therapeutischen und/oder desynchronisierenden Effekt erzielen würde, wenn man die Reize 22 während der gesamten Stimulationsdauer, d. h. während des ersten und des zweiten Zeitintervalls, applizieren würde. Gemäß einer Ausgestaltung ist die untere Grenze für die Reizstärke im zweiten Stimulationsmodus größer als das 1,3-fache der vorgegebenen Reizstärke und beträgt das 1,5- oder 1,7-fache der vorgegebenen Reizstärke. Sofern es sich bei den Reizen 22 um elektrische stromgesteuerte Reize 22 handelt, ist die Reizstärke durch die Stromstärke der Reize 22 gegeben. Im Fall von optischen Reizen 22 kann die Reizstärke die Lichtstärke der Reize 22 sein.

Bei der hier beschriebenen Zwei-Stufen-CR-Stimulation wird die Reizstärke ohne Verlust oder Einschränkung der Wirksamkeit aufdosiert. Während der ersten Stufe, d. h. im ersten Stimulationsmodus, genügt eine unterschwellige Reizstärke, wodurch ungewünschte Effekte deutlich reduziert werden können. Durch die Stimulation im ersten Stimulationsmodus wird die stimulierte Neuronenpopulation in einen Zustand gebracht, in dem sie für die nachfolgend in der zweiten Stufe durchgeführte Stimulation im zweiten Stimulationsmodus deutlich empfänglicher ist. Die Zwei-Stufen-CR-Stimulation ermöglicht folglich eine verbesserte Stimulationswirkung bei gleichzeitig reduzierten Nebenwirkungen und anderen unerwünschten Effekten.

Das zugrundeliegende Wirkprinzip der Zwei-Stufen-CR-Stimulation, nämlich die Verstärkung der desynchronisierenden Wirkung der Stimulation mit langsam variierender Sequenz durch vorgeschaltete Stimulation mit rasch variierender Sequenz, gilt nicht nur bei unterschwelliger Reizstärke der Stimulation mit rasch variierender Sequenz. Vielmehr ist die Wirkung der Zwei-Stufen-CR-Stimulation bei überschwelliger erster Stufe zumindest tendenziell besser als alle sonstigen Varianten der CR-Stimulation gleicher Intensität und Dauer. Für den Fall der ersten Stufe mit überschwelliger Reizstärke entfällt aber der besondere Vorteil, dass durch die Verwendung der unterschwelligen Stimulation Nebenwirkungen und andere unerwünschte Effekte vermieden oder zumindest reduziert werden können.

Sofern für die Zwei-Stufen-CR-Stimulation die in Fig. 7 dargestellte Vorrichtung 2 eingesetzt wird, also eine "closed loop"-Stimulation durchgeführt wird, kann die Steuereinheit 10 anhand der in Reaktion auf die Applikation der Reize 22 von der Messeinheit 13 aufgenommenen Messsignale 23 den Stimulationserfolg überprüfen.

Sobald insbesondere anhand der Messsignale 23 eine ausgeprägte Desynchronisation bzw. akute klinische Befundbesserung bzw. eine ausgeprägte Besserung der Befindlichkeit des Patienten festgestellt worden ist, kann, insbesondere mit Hilfe der Steuereinheit 10, von dem ersten Stimulationsmodus in den zweiten Stimulationsmodus umgeschaltet werden. Insbesondere kann eine mit der Steuereinheit 10 gekoppelte Eingabeeinheit vorgesehen sein, die von dem Patienten und/oder dem behandelnden Arzt bedient werden kann, und mit der vom ersten Stimulationsmodus in den zweiten Stimulationsmodus umgeschaltet werden kann.

Der Stimulationserfolg kann insbesondere mittels eines Schwellwertvergleichs überprüft werden. Je nachdem welche Signale zur Ermittlung des Stimulationserfolgs herangezogen werden, ergeben sich unterschiedliche Schwellwertvergleiche. Wird z. B. die krankhafte neuronale Synchronisation über die Sensoren der Messeinheit 13, z. B. EEG-Elektroden oder Tiefelektroden (als LFP-Signal), gemessen, reicht erfahrungsgemäß die Absenkung der Synchronisation um einen vorgegebenen Wert, z. B. um mindestens 20%, im Vergleich zur Situation ohne Stimulation, um einen ausreichenden Stimulationserfolg festzustellen und vom ersten in den zweiten Stimulationsmodus zu wechseln. Es können aber größere Werte, z. B. 50% und mehr, gewählt werden, um länger im ersten Stimulationsmodus und somit mit geringerer Reizstärke zu stimulieren.

Die klinische Befundbesserung wird anhand typischer, dem Fachmann bekannter Änderungen von klinischen Scores bzw. Fragebögen bestimmt. Hierzu werden z. B. die aus der Literatur bekannten Werte Delta S für einen "minimal clinically relevant change" (minimale klinisch relevante Änderung) oder auch größere Werte, z. B. 2 x Delta S, verwandt.

Zusätzlich zu der oben beschriebenen Regelung, die das Umschalten von dem ersten in den zweiten Stimulationsmodus bestimmt, kann eine weitere Regelung vorgesehen sein, die auf einer langsameren Zeitskala agiert. Wenn sich ein Therapieerfolg über einen vordefinierten Zeitraum, z. B. 1 Stunde, eingestellt hat, wird die Stimulation abgeschaltet. Der Therapieerfolg wird hierbei wie oben beschrieben gemessen, wobei die Schwellenwerte für einen ausreichenden Therapieerfolg vom Anwender voreingestellt werden können, z. B. eine Absenkung der anfänglichen Synchronisation um 80%. Wenn diese Schwellenwerte für eine vordefinierte Dauer, z. B. 60 s, wieder überschritten werden und/oder der Patient eine nicht mehr hinreichend gebesserte Befindlichkeit meldet, wird die Zwei-Stufen-CR-Stimulation wie oben beschrieben neu gestartet.

Mit Hilfe der Messeinheit 13 der Vorrichtung 2 können Werte für die Längen des ersten Zeitintervalls und des zweiten Zeitintervalls für einen jeweiligen Patienten abgeschätzt werden, die benötigt werden, um den gewünschten Stimulationserfolg zu erzielen. Anschließend können diese Informationen für eine Anwendung mit der Vorrichtung 1, die über keine Messeinheit verfügt, verwendet werden. Grundsätzlich können die Längen des ersten und des zweiten Zeitintervalls im Minuten- oder Stundenbereich liegen.

Weiterhin lässt sich mit Hilfe der Messeinheit 13 gemäß einer Ausgestaltung die vorgegebene Reizstärke bestimmen, aus der sich die obere bzw. untere Grenze für die Reizstärken im ersten und zweiten Stimulationsmodus ergibt. Auch diese Information kann anschließend bei einer Anwendung mit der Vorrichtung 1 genutzt werden. Zur Bestimmung der vorgegebenen Reizstärke wird die Stimulationseinheit 11 z. B. im ersten Stimulationsmodus betrieben und die Stärke der Reize 22 wird ausgehend von Null solange erhöht, bis sich ein Akuteffekt einstellt, d. h. eine Verringerung der Synchronisation der stimulierten Neuronenpopulation, die jedoch nach der Beendigung der Stimulation wieder verschwindet. Aus der so gewonnenen Reizstärke kann die vorgegebene Reizstärke abgeleitet werden, indem die vorgegebene Reizstärke beispielsweise aus einem Bereich ausgewählt wird, dessen untere Grenze die Reizstärke, bei der eine Verringerung der Synchronisation der stimulierten Neuronenpopulation einsetzt, darstellt und dessen obere Grenze z. B. das 1,1-fache der vorstehenden Reizstärke ist.

Vorstehend wurden die vier verschiedenen, in den Teilabbildungen von Fig. 9 illustrierten CR-Stimulationsvarianten nur beispielhaft anhand der Gruppe 1 und deren Untergruppen 1_1, 1_2 und 1_3 erläutert. Die hierin beschriebenen CR-Stimulationsformen können in entsprechender Weise auf andere Gruppen von Stimulationselementen und deren Untergruppen sowie insbesondere die in Fig. 8 gezeigten Gruppen 2 sowie 3 und deren Untergruppen angewandt werden.

Es hat sich überraschenderweise gezeigt, dass die Stimulationsergebnisse bei räumlich inhomogenen neuronalen Synchronisationsprozessen besonders gut sind, wenn über die jeweiligen Gruppen von Stimulationselementen mit der geeignetsten CR-Variante stimuliert wird. Aus diesem Grund wird bei der Mehrsegment-Neurostimulation die CR-Stimulation für jede Gruppe von Stimulationselementen einzeln möglichst optimal appliziert.

Kommt es zu Nebenwirkungen bei Reizung über eine bestimmte Gruppe von Stimulationselementen und/oder ist die zugehörige Nebenwirkungsschwelle erniedrigt, wird mit geringerer Amplitude und vorzugsweise Zwei-Stufen-CR-Stimulation gereizt. Es kann auch die intra burst-Frequenz, also die Frequenz innerhalb der Pulszüge eines Reizes 22 vermindert und gegebenenfalls die Amplitude der Reize 22 kompensatorisch erhöht werden.

Sollte die in Fig. 7 dargestellte Vorrichtung 2 im Sinne einer "closed loop"-Variante zum Einsatz kommen, kann es vorteilhaft sein, die Frequenz $f_{stim}$ (=$1/T_{stim}$) einer jeweiligen Gruppe an die dominante Frequenz des von der entsprechenden Gruppe stimulierten Teilbereichs des Zielareals anzupassen. Die dominante Frequenz kann in dem jeweiligen Teilbereich z. B. durch kontinuierliche oder intermittente Messungen kontinuierlich bzw. regelmäßig gemessen werden und die Frequenz $f_{stim}$ kann dementsprechend angepasst werden.

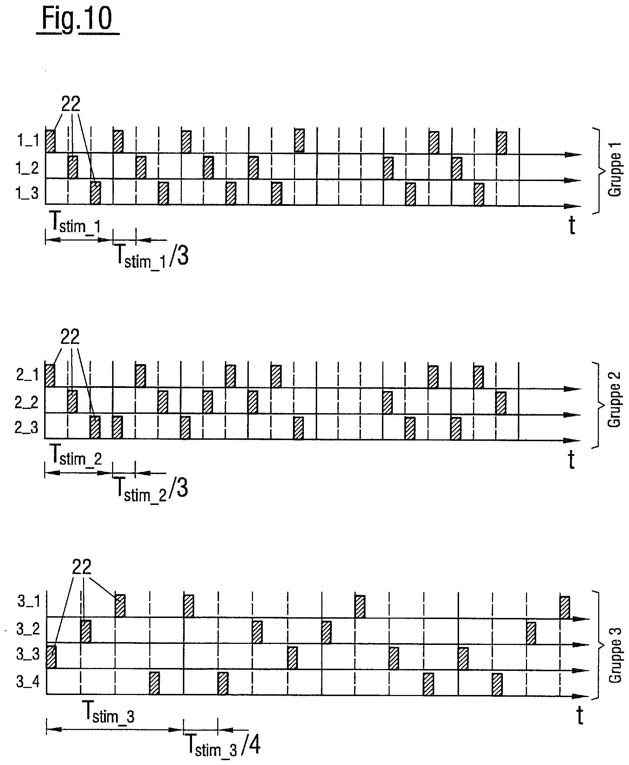

Auf diese Weise können über verschiedene Gruppen von Stimulationselementen unterschiedliche CR-Varianten mit jeweils gleichen oder unterschiedlichen Frequenzen $f_{stim}$ appliziert werden. Beispielhaft ist eine derartige Stimulation in Fig. 10 gezeigt. Hier applizieren die Gruppen 1, 2 und 3 der in Fig. 8 dargestellten Vielkanal-Elektrode 40 unterschiedliche CR-Varianten. In Fig. 10 sind untereinander die von den jeweiligen Untergruppen der Gruppen 1, 2 und 3 erzeugten Reize 22 gegen die Zeit t aufgetragen.

Bei der klinischen Austestung der einzelnen Gruppen mittels Hochfrequenz-Dauerstimulation und/oder der Testung mittels CR-Applikation ergab sich, dass bei dem Patienten bei Stimulation über die Gruppen 2 und 3 Nebenwirkungen auftraten und/oder die Nebenwirkungsschwelle verringert war. Deswegen wird über die Gruppen 2 und 3 jeweils eine Zwei-Stufen-CR-Stimulation appliziert, wie sie oben im Zusammenhang mit der vierten Teilabbildung von Fig. 9 erläutert wurde. Bei der Zwei-Stufen-CR-Stimulation wird in der ersten Stufe, d. h. im ersten Stimulationsmodus, eine CR-Stimulation mit schnell variierender Sequenz appliziert, in der zweiten Stufe, d. h. im zweiten Stimulationsmodus, hingegen eine CR-Stimulation mit langsam variierender Sequenz. Zu Illustrationszwecken ist in Fig. 10 für die Gruppen 2 und 3 jeweils nur die erste Stufe, die CR-Stimulation mit schnell variierender Sequenz, dargestellt. In dieser Stufe wird in dem vorliegenden Beispiel von einem Zyklus zum nächsten die Sequenz randomisiert.

Bei der Reizung über die Gruppe 1 kam es bei dem Patienten zu keinen Nebenwirkungen und/oder keiner erniedrigten Nebenwirkungsschwelle. Deswegen wird über die Gruppe 1 eine CR-Stimulation mit langsam variierender Sequenz verabreicht, wie sie oben im Zusammenhang mit der dritten Teilabbildung von Fig. 9 erläutert wurde.

Ferner wurde für jeden von den Gruppen 1, 2 und 3 stimulierten Teilbereich die dominante Frequenz der pathologischen neuronalen Aktivität gemessen. Die Periodenlängen $T_{stim}$ der Gruppen 1, 2 und 3 wurden an die jeweils gemessene dominante mittlere Frequenz angepasst. Die entsprechenden Periodenlängen der Gruppen 1, 2 und 3 sind in Fig. 10 mit $T_{stim\_1}$, $T_{stim\_2}$ bzw. $T_{stim\_3}$ bezeichnet. Allgemein kann beispielsweise die Periodenlänge $T_{stim\_i}$ einer Gruppe i dem Inversen der in dem von der Gruppe i stimulierten Teilbereich gemessenen dominanten mittleren Frequenz der pathologischen neuronalen Aktivität gleichgesetzt werden. Gemäß einer Ausgestaltung kann die Periodenlänge $T_{stim\_i}$ in einem Bereich von 10 ms oder 20 ms oder 50 ms oder 100 ms oder 200 ms oder 2000 ms um das Inverse der in dem von der Gruppe i stimulierten Teilbereich gemessenen dominanten mittleren Frequenz der pathologischen neuronalen Aktivität gewählt werden.

Die über die Gruppen 1 und 2 applizierte CR-Stimulation hat in dem in Fig. 10 gezeigten Ausführungsbeispiel dieselbe Stimulationsperiode, d. h. $T_{stim\_1} = T_{stim\_2}$, da die über die Gruppen 1 und 2 zu stimulierenden neuronalen Synchronisationsprozesse dieselbe bzw. eine ähnliche zeitlich gemittelte dominante Frequenz haben. Der über die Gruppe 3 zu stimulierende Synchronisationsprozess hat eine kleinere dominante mittlere Frequenz und wird dementsprechend wird mit der längeren Stimulationsperiode $T_{stim\_3}$ gereizt. Auf diese Weise werden die den unterschiedlichen Gruppen 1, 2 und 3 zugeordneten neuronalen Synchronisationsprozesse spezifisch stimuliert - gemäß anatomischer Verteilung (durch Wahl der Elektroden und Wahl der Reizamplitude des jeweiligen Kontakts), dynamischer Charakteristika (insbesondere hinsichtlich der dominanten Frequenz, welche die jeweilige Periodenlänge $T_{stim}$ bestimmt) sowie Nebenwirkungsprofil.

Allgemein erzeugen bei der vorstehend beschriebenen Stimulationsform mindestens eine erste Gruppe von Stimulationselementen und eine zweite Gruppe von Stimulationselementen Sequenzen von Reizen repetitiv in einem jeweiligen Zeitraster, das aus aufeinanderfolgenden Zyklen besteht. Die von der ersten Gruppe erzeugten Sequenzen von Reizen unterscheiden sich von den von der zweiten Gruppe erzeugten Sequenzen von Reizen.

Der Unterschied zwischen den von den beiden Gruppen erzeugten Sequenzen kann in der Anzahl nacheinander generierter Sequenzen, nach denen die Reihenfolge der Stimulationselemente innerhalb einer Sequenz variiert wird, liegen. Beispielsweise kann die erste Gruppe eine Zwei-Stufen-CR-Stimulation durchführen, wie sie beispielhaft in der vierten Teilabbildung von Fig. 9 dargestellt ist. Für die erste Gruppe ist in diesem Fall im ersten Stimulationsmodus die Reihenfolge, in welcher die Stimulationselemente innerhalb einer Sequenz die Reize erzeugen, für höchstens 5 nacheinander generierte Sequenzen konstant und wird danach variiert und im zweiten Stimulationsmodus ist die Reihenfolge, in welcher die Stimulationselemente innerhalb einer Sequenz die Reize erzeugen, für mindestens 25 nacheinander generierte Sequenzen konstant und wird danach variiert. Ferner kann beispielsweise die zweite Gruppe eine CR-Stimulation mit einer langsamen Variation der Sequenzen durchführen, wie sie beispielhaft in der dritten Teilabbildung von Fig. 9 dargestellt ist. Die Reihenfolge, in welcher die Stimulationselemente der zweiten Gruppe innerhalb einer Sequenz die Reize erzeugen, wird dann für mindestens 20 nacheinander generierte Sequenzen konstant gehalten und danach variiert.

Ferner können weitere Gruppen von Stimulationselementen vorgesehen sein, die beispielsweise gleiche Sequenzen von Reizen wie die erste oder zweite Gruppe oder auch unterschiedliche Sequenzen von Reizen erzeugen. Beispielsweise kann eine dritte Gruppe von Stimulationselementen eine CR-Stimulation durchführen, wie sie beispielhaft in der zweiten Teilabbildung von Fig. 9 dargestellt ist, und insbesondere eine vierte Gruppe von Stimulationselementen kann eine CR-Stimulation durchführen, wie sie beispielhaft in der ersten Teilabbildung von Fig. 9 dargestellt ist.

Zusätzlich oder alternativ können sich die von der ersten, der zweiten und eventuell weiteren Gruppen erzeugten Sequenzen von Reizen in der Dauer der jeweiligen Zyklen unterscheiden. Dies ist beispielhaft in Fig. 10 gezeigt. Hier weist die Gruppe 1 eine Zyklusdauer $T_{stim\_1}$ auf und die Gruppe 3 hat eine Zyklusdauer $T_{stim\_3}$, wobei $T_{stim\_1} < T_{stim\_3}$ gilt.

Statt der CR-Stimulation können aber auch andere desynchronisierende Stimulationsverfahren zum Einsatz kommen.

Überraschenderweise hat sich gezeigt, dass die kombinierte Anwendung von CR-Stimulation mit Hochfrequenz-Dauerstimulation in jeweils unterschiedlichen Segmenten vorteilhaft sein kann. Die Hochfrequenz-Dauerstimulation, bei welcher Pulse periodisch mit einer Wiederholfrequenz von mindestens 100 Hz verabreicht werden, hat - einzeln appliziert - typischerweise keine lang anhaltenden desynchronisierenden Effekte. Wenn nun bei einem räumlich inhomogenen neuronalen Synchronisationsprozess z. B. die Messung ergibt, dass die dominanten Frequenzen der unterschiedlichen Teilbereiche zeitlich stark variieren und/oder sogar obendrein die Grenzen der jeweiligen Teilbereiche zeitlich variieren, ist es vorteilhaft, manche Teilbereiche durch Beaufschlagung mit Hochfrequenz-Dauerstimulation vorübergehend quasi auszuschalten und während dessen die nicht ausgeschalteten Teilbereiche mit CR-Stimulation zu behandeln. Hierzu wird dann über alle oder einige oder nur einen Kontakt einer Gruppe eine Hochfrequenz-Dauerstimulation appliziert, während die anderen Gruppen mit jeweils individuell angepasster CR-Stimulation gereizt werden. Dieses Stimulationsprinzip kann quasi iterativ, d. h. Schritt für Schritt, auf den nach erfolgreicher CR-Stimulation übrig bleibenden neuronalen Synchronisationsprozess angewendet werden. Für jeden Schritt dieses iterativen Verfahrens werden zur Stimulation geeignete Gruppen bzw. Segmente ausgewählt, bis am Schluss keine bzw. keine klinisch störende neuronale Synchronisation übrig geblieben ist.

Der Erfolg der CR-Stimulation kann bei diesem iterativen Vorgehen klinisch erfasst und/oder im Rahmen einer "closed loop"-Variante und/oder durch gelegentlich, also nicht kontinuierlich verwendete Sensoren überprüft werden. Die objektive Abschätzung des Stimulationserfolgs wird durch die Absenkung der Amplitude der krankhaften neuronalen Synchronisation bestimmt.

Die kombinierte Anwendung von CR-Stimulation und Hochfrequenz-Dauerstimulation kann auch vorteilhaft sein, wenn, z. B. aus medizinischer Indikation, keine Signale über weitere implantierte Elektroden abgeleitet werden können und sich der Behandlungseffekt nicht hinreichend schnell einstellt.

Ziel der iterativen Vielkanal-Stimulation ist es, kleinere, durch Reizung besser kontrollierbare Bereiche mit CR-Stimulation zu behandeln. Z. B. kann sich die zeitliche Variabilität der dominanten Frequenz des zu behandelnden Bereichs dadurch verringern, dass der zu stimulierende Teilbereich zu nur einem Abschnitt einer Gliedmaße gehört und somit über das propriozeptive Feedback (also über neuronale Signale der Eigenwahrnehmung der Gliedmaße) nur mit einer vorwiegend mechanisch bedingten Eigenfrequenz angetrieben wird.

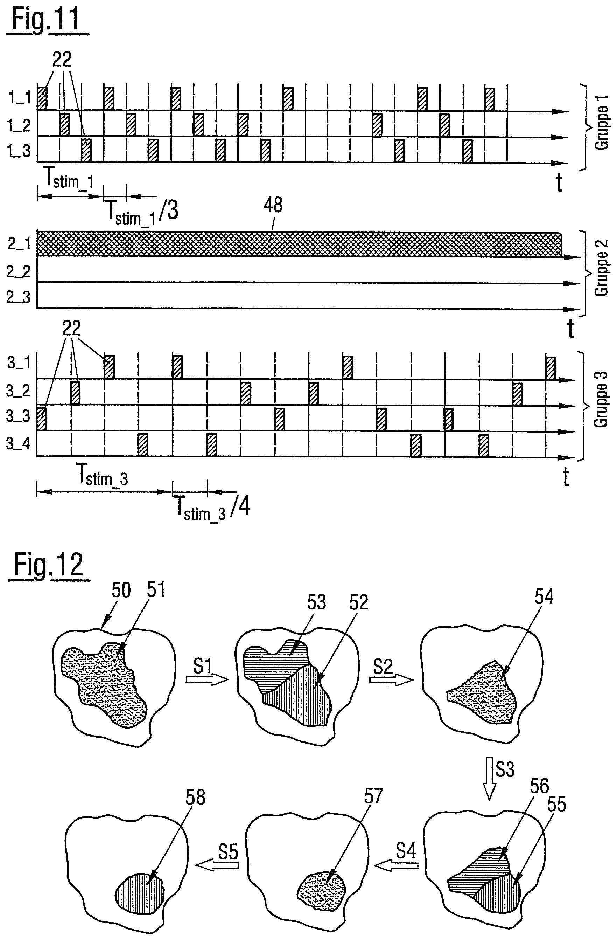

Fig. 11 zeigt eine mit der in Fig. 8 dargestellten Vielkanal-Elektrode 40 applizierte Stimulation, bei der über die Gruppen 1 und 3 eine an den jeweils stimulierten Teilbereich des Zielareals angepasste CR-Stimulation appliziert wird. Die von den Gruppen 1 und 3 applizierten CR-Stimulationen entsprechen den CR-Stimulationen aus Fig. 10.

Im Vergleich zu Fig. 10 wird in Fig. 11 über die Gruppe 2 nicht mit CR-Stimulation, sondern mit Standard-Hochfrequenz-Dauerstimulation gereizt. Hierbei wird dauerhaft, d. h. ohne Pause, ein periodischer Pulszug mit einer Pulsrate, d. h. einer Wiederholfrequenz der Pulse, von mehr als 100 Hz appliziert. Die Pulse des Pulszugs können insbesondere identisch sein. In Fig. 11 ist die Standard-Hochfrequenz-Dauerstimulation durch einen durchgehenden horizontalen Balken 48 gekennzeichnet. Die Standard-Hochfrequenz-Dauerstimulation kann über eine einzige Untergruppe oder mehrere Untergruppen oder alle Untergruppen der jeweiligen Gruppe appliziert werden. In Fig. 11 wird beispielhaft nur über die Untergruppe 2_1 die Standard-Hochfrequenz-Dauerstimulation appliziert. Eine "dauerhafte" Applikation des periodischen Pulszugs bedeutet hier, dass der periodische Pulszug zumindest während der Applikation der parallelen CR-Stimulation durch eine andere Gruppe appliziert wird.

Die in Fig. 11 dargestellte Stimulationsform wird angewandt, da es sich als vorteilhaft erweisen kann, räumlich ausgedehnte neuronale Synchronisationsprozesse mit qualitativ unterschiedlichen Stimulationsmethoden zu reizen. Durch die Standard-Hochfrequenz-Dauerstimulation können die zugehörigen synchronen Bereiche inhibiert und/oder blockiert werden, wodurch der therapeutische Umbau der synaptischen Konnektivität in den beiden anderen neuronalen Bereichen deutlich schneller vonstatten geht.

Hierzu wird dann über alle oder einige oder nur einen Kontakt einer Gruppe eine Hochfrequenz-Dauerstimulation appliziert, während die anderen Gruppen eine CR-Stimulation applizieren. Die CR-Stimulation kann dabei jeweils individuell angepasst sein, insbesondere kann aus den im Zusammenhang mit Fig. 9 beschriebenen vier verschiedenen CR-Reizfolgen gewählt werden. Dieses Stimulationsprinzip kann quasi iterativ, d. h. Schritt für Schritt, auf den nach erfolgreicher CR-Stimulation übrig bleibenden neuronalen Synchronisationsprozess angewendet werden. Für jeden Schritt dieses iterativen Verfahrens werden zur Stimulation geeignete Gruppen bzw. Segmente ausgewählt, bis am Schluss keine bzw. keine klinisch störende neuronale Synchronisation übrig geblieben ist.

Fig. 12 veranschaulicht beispielhaft das Prinzip der iterativen Vielkanal-Stimulation. Die schematische Abbildung zeigt ein Zielareal 50 im Gehirn und/oder Rückenmark eines Patienten, in dem sich in einem räumlich umschriebenen Bereich 51 ein bezüglich seiner funktionalen Charakteristika inhomogener neuronaler Synchronisationsprozess befindet. In Schritt S1 wird ein Teilbereich 52 mit Hilfe einer ersten Gruppe von Stimulationselementen mit einer Standard-Hochfrequenz-Dauerstimulation beaufschlagt, während ein Teilbereich 53 mit Hilfe einer zweiten Gruppe von Stimulationselementen mit CR-Stimulation gereizt wird. Durch die CR-Stimulation verschwindet schließlich die krankhafte neuronale Synchronisation im Teilbereich 53, so dass nur noch in einem Teilbereich 54 eine krankhafte neuronale Synchronisation übrig bleibt (vgl. Schritt S2). In Schritt S3 wird ein Teilbereich 56 mit Hilfe einer dritten Gruppe von Stimulationselementen mit CR-Stimulation gereizt und ein Teilbereich 55 wird mit Hilfe einer vierten Gruppe von Stimulationselementen mit Standard-Hochfrequenz-Dauerstimulation gereizt, wobei die dritte und die vierte Gruppe jeweils zumindest einige der Stimulationselemente der ersten Gruppe enthalten. Übrig bleibt schließlich nur noch krankhafte neuronale Synchronisation in einem Teilbereich 57 (vgl. Schritt S4). In Schritt S5 wird schließlich ein Teilbereich 58 mit Hilfe einer fünften Gruppe von Stimulationselementen ausschließlich mittels CR-Stimulation gereizt, so dass am Ende keine oder kaum krankhafte neuronale Synchronisation übrig bleibt.

Die zeitgleiche Stimulation eines Teilbereichs des Zielareals mit Standard-Hochfrequenz-Dauerstimulation und eines oder mehrerer anderer Teilbereiche mit CR-Stimulation kann beispielsweise so lange erfolgen, bis anhand der von der Messeinheit 13 aufgenommenen Messsignale 23 festgestellt wird, dass der Synchronisationsgrad der mit den CR-Reizfolgen stimulierten Neuronen im Vergleich zum Zustand vor der CR-Stimulation um mindestens einen vorgegebenen Schwellwert reduziert wurde. Die daraus gewonnene Zeitdauer kann für eine Anwendung mit der Vorrichtung 1, die über keine Messeinheit verfügt, verwendet werden. In diesem Fall kann die zeitgleiche Stimulation eines Teilbereichs des Zielareals mit Standard-Hochfrequenz-Dauerstimulation und eines oder mehrerer anderer Teilbereiche mit CR-Stimulation beispielsweise nach einer vorgegebenen Zeitdauer beendet werden. Grundsätzlich kann die Dauer einer derartigen Stimulation im Minuten- oder Stundenbereich liegen.

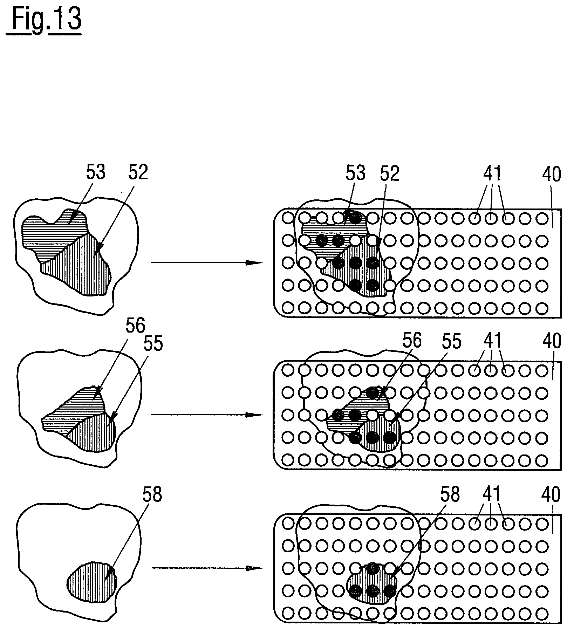

Fig. 13 ist eine schematische Abbildung der für die iterative Vielkanal-Stimulation aus Fig. 10 beispielhaft verwendeten Kontakte 41 einer Vielkanal-Elektrode 40 zur Stimulation der Teilbereiche 52 und 53 (vgl. obere Teilabbildung), der Teilbereiche 55 und 56 (vgl. mittlere Teilabbildung) und des Teilbereichs 58 (vgl. untere Teilabbildung). Die zur Stimulation herangezogenen Kontakte 41 sind jeweils dunkel eingefärbt und verdeutlichen die ersten bis fünften Gruppen von Stimulaitonselementen.

Die einzelnen Komponenten der Vorrichtungen 1 und 2, insbesondere die Steuereinheit 10, die Stimulationseinheit 11 und/oder die Messeinheit 13, können baulich voneinander getrennt sein. Die Vorrichtungen 1 und 2 können daher auch als Systeme aufgefasst werden. Zur Durchführung ihrer Aufgaben kann die Steuereinheit 10 einen Prozessor, z. B. einen Mikrocontroller, enthalten. Die hierin beschriebenen Stimulationsverfahren können als Software-Code in einem der Steuereinheit 10 zugeordneten Speicher abgelegt sein.

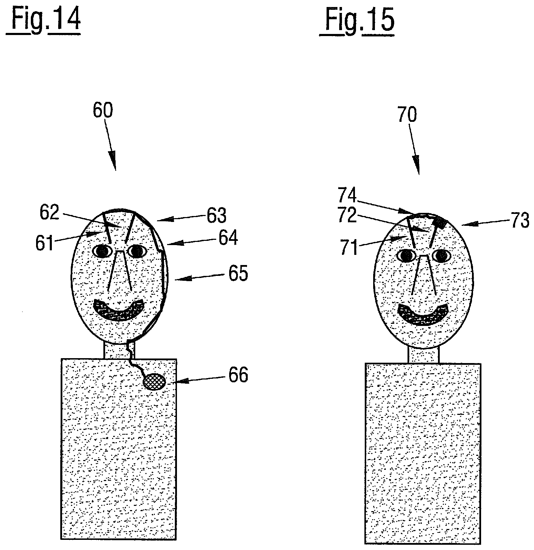

Fig. 14 zeigt schematisch eine Vorrichtung 60 zur invasiven elektrischen Stimulation von Neuronen mit einer krankhaft synchronen und oszillatorischen neuronalen Aktivität gemäß einer Ausführungsform der Erfindung. Die Vorrichtung 40 umfasst zwei Vielkanal-Elektroden 61, 62, die in das Gehirn des Patienten implantiert sind und über Kabel 63 mit einem Konnektor 64 verbunden sind. Der Konnektor 64 wiederum ist über ein Kabel 65 mit einer Steuereinheit 66 verbunden. Die Vorrichtung 60 kann die Funktionen der oben beschriebenen Vorrichtungen 1 und 2 aufweisen.

Fig. 15 zeigt schematisch eine weitere Vorrichtung 70 zur invasiven elektrischen Stimulation von Neuronen mit einer krankhaft synchronen und oszillatorischen neuronalen Aktivität gemäß einer Ausführungsform der Erfindung. In gleicher Weise wie die Vorrichtung 60 umfasst die Vorrichtung 70 zwei implantierte Vielkanal-Elektroden 71, 72. Die Vorrichtung 70 umfasst ferner einen im Bohrloch implantierten Generator 73, der mit der Vielkanal-Elektrode 72 direkt verbunden ist. Die Vielkanal-Elektrode 71 ist mit dem Generator 73 über ein Kabel 74 verbunden.

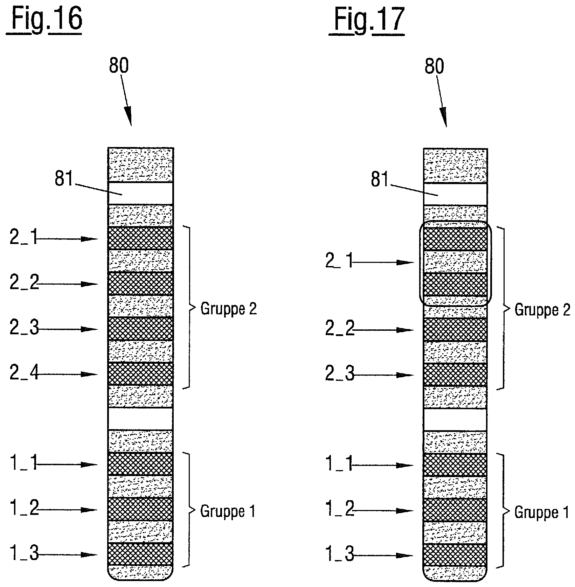

Die erfindungsgemäße Vorrichtung kann über Vielkanal-Elektroden, wie sie z. B. in den Figuren 4 und 8 dargestellt sind, und/oder über Mehrkanal-Elektroden verfügen. Für Vielkanal- und Mehrkanal-Elektroden können Stimulations- bzw. Messkontakte unterschiedlicher Geometrie genutzt werden. Kontakte unterschiedlicher Geometrie können auch baulich vereint sein. Beispielhaft ist eine Mehrkanal-Elektrode 80 mit ringförmigen Kontakten 81 in Fig. 16 und 17 dargestellt. Über dunkel markierte Kontakte 81 wird hier beispielhaft stimuliert, während über die weiß markierten Kontakte 81 nicht stimuliert wird.

In Fig. 16 umfasst die Gruppe 1 die Untergruppen 1_1, 1_2 und 1_3 und die Gruppe 2 umfasst die Untergruppen 2_1, 2_2, 2_3 und 2_4, wobei in diesem Bespiel jede Untergruppe aus genau einem Kontakt 81 besteht. In Fig. 17 besteht die Untergruppe 2_1 aus zwei Kontakten 81. Über die Gruppen 1 und 2 können die in dieser Anmeldung beschriebenen Stimulationen appliziert werden.

Implantierbare Stimulationseinheiten für die optische Stimulation von neuronalem Gewebe sind bekannt. Beispielsweise kann eine Lichtquelle, wie z. B. ein Laser, eine Laserdiode oder eine LED, einen Lichtstrahl erzeugen, der mit Hilfe einer Lichteinkopplung auf die Eingänge eines aus mehreren Lichtleitern bestehenden Faserbündels verteilt wird. Eine Steuereinheit gibt dabei z. B. vor, zu welchem Zeitpunkt ein einzelner Lichtpuls oder ein Zug von Lichtpulsen in welche Faser des Faserbündels eingekoppelt wird. Die Auskopplungspunkte der einzelnen Fasern des Fasernbündels, d. h. die Enden der Fasern, liegen an verschiedenen Stellen im Zielgebiet im Gehirn und/oder Rückenmark des Patienten. Das Licht stimuliert somit unterschiedliche Orte des Zielgebiets in einer durch die Steuereinheit vorgegebenen zeitlichen Abfolge. Es können allerdings auch andere implantierbare Stimulationseinheiten verwendet werden, die sich zur direkten optischen Stimulation von neuronalem Gewebe eignen.

Wie oben beschrieben bewirken die Reize 22 bei der CR-Stimulation ein Zurücksetzen, einen sogenannten Reset, der Phase der neuronalen Aktivität der stimulierten Neuronen. Mit Hilfe der von der Messeinheit 13 aufgenommenen Messsignale 23 kann die Phasenrücksetzung der einzelnen Reize 22 überprüft werden. Eine derartige Untersuchung kann vor der eigentlichen therapeutischen Neurostimulation vorgenommen werden.

Dazu wird über einen Sensor der Messeinheit 13 ein Signal gemessen, welches die Aktivität der über den j-ten Stimulationskanal stimulierten Subpopulation hinreichend repräsentiert. Man erhält dieses Signal entweder direkt von der Subpopulation über eine nicht-invasive Messung, z. B. über EEG- oder MEG-Elektroden, oder eine invasive Messung, z. B. über implantierte Elektroden, als Oberflächen-EEG oder als lokales Feldpotential und/oder Ableitungen von Gruppen von einzelnen Neuronen (multi unit activity = MUA) über Tiefenelektroden. Das Signal kann auch indirekt über die Messung einer mit der Aktivität der stimulierten Subpopulation korrelierten Größe ermittelt werden. Hierzu eignen sich z. B. EEG-/MEG-/LFP-/MUA-Signale der neuronalen Aktivität einer mit dieser Subpopulation eng gekoppelten anderen Neuronenpopulation oder zugehörige Elektromyographie-, Akzelerometer- oder Gyroskop-Signale.

Da neuronale Signale typischerweise rhythmische Aktivität in unterschiedlichen Frequenzbändern enthalten, ist es in solchen Fällen vorteilhaft, z. B. mittels Bandpassfilterung oder wavelet-Analyse oder empirical mode decomposition das Signal $x_j(t)$, welches die pathologische oszillatorische Aktivität der vom j-ten Stimulationskanal stimulierten Subpopulation repräsentiert, zu ermitteln.

Ein nur wenig aufwändiges Vorgehen, um eine Phasenrücksetzung zu überprüfen, besteht darin, die gemittelte Reizantwort zu bestimmen. Hierzu wird zu den Zeiten $\tau_1$, $\tau_2$, ..., $\tau_l$ ein Reiz mit identischen Reizparametern appliziert. Die Abstände zwischen den einzelnen Reizen $\tau_{k+1} - \tau_k$ sollten hinreichend groß und randomisiert, also nicht konstant sein, um Einschwingvorgänge zu vermeiden (vgl. P. A. Tass: Transmission of stimulus-locked responses in two coupled phase oscillators. Phys. Rev. E 69, 051909-1-24 (2004)). Typischerweise sollten die Abstände $\tau_{k+1} - \tau_k$ im Bereich von mindestens dem Zehnfachen, besser dem Hundertfachen der mittleren Periode des pathologischen Oszillation liegen. Die über alle I Test-Reize gemittelte Reizantwort wird gemäß folgender Gleichung berechnet:

$$\overline{x}_j(t) = \frac{1}{l}\sum_{k=1}^{l} x_j(\tau_k + t) \tag{1}$$

Sofern die Abstände $\tau_{k+1} - \tau_k$ zwischen den einzelnen Reizen hinreichend groß sind, erhält man im prä-Stimulus-Bereich, d. h. im Bereich vor der Applikation eines jeweiligen Reizes, keine gemittelte Reizantwort (vgl. P. A. Tass: Transmission of stimulus-locked responses in two coupled phase oscillators. Phys. Rev. E 69, 051909-1-24 (2004)). Eine Phasenrücksetzung kann festgestellt werden, wenn eine gemittelte Reizantwort detektiert werden kann, d. h., wenn sich im post-Stimulus-Bereich, d. h. im Bereich für t > 0, wobei t = 0 den Anfangszeitpunkt des jeweiligen Reizes darstellt, eine von Null verschiedene Reizantwort findet. Dies kann durch visuelle Inspektion ermittelt werden. Man kann dies auch von der Vorrichtung 2, insbesondere der Steuereinheit 10, durchführen lassen, indem man die prä-Stimulus-Verteilung von $\overline{x}_j(t)$ oder $|\overline{x}_j(t)|$ betrachtet und einen charakteristischen Schwellwert, z. B. die 99te Perzentile der prä-Stimulus-Verteilung von $|\overline{x}_j(t)|$ oder schlicht deren Maximum bestimmt. Wenn nun z. B. der Betrag der post-Stimulus-Antwort prinzipiell oder für eine vorgegebene Mindestdauer, z. B. 20 ms, diesen charakteristischen Schwellenwert übersteigt, liegt eine von Null verschiedene gemittelte Antwort vor. In diesem Fall kann eine Phasenrücksetzung vorliegen. D. h., die Reizstärke müsste so lange erhöht werden, bis die post-Stimulus-Antwort sich von einer Nulllinie unterscheidet. Neben dem hier vorgestellten einfachen, aber in der Praxis bewährten Verfahren können auch andere, dem Fachmann bekannte statistische Tests zur Signalanalyse herangezogen werden.

Eine genauere, aber aufwändigere Variante zur Untersuchung, ob die Reize eine Phasenrücksetzung bewirken, bietet die Analyse der Phase. Hierzu wird die Phase $\psi_j(t)$ von $x_j(t)$ bestimmt. Dies erfolgt mittels Hilbert-Transformation aus dem mittels Bandpassfilterung bzw. empirical mode decomposition bestimmten Signal, welches die pathologische oszillatorische Aktivität repräsentiert. Die empirical mode decomposition ermöglicht im Vergleich zur Bandpassfilterung eine parameterunabhängige Bestimmung physiologisch relevanter Moden in verschiedenen Frequenzbereichen (vgl. N. E. Huang et al.: The empirical mode decomposition and the Hilbert spectrum for nonlinear and non-stationary time series analysis. Proc. R. Soc. A: Math. Phys. Eng. Sci. 454:903-995 (1998)). Die Kombination von empirical mode decomposition mit nachfolgender Hilbert-Transformation wird als Hilbert-Huang-Transformation bezeichnet (vgl. N. E. Huang et al.: A confidence limit for the empirical mode decomposition and Hilbert spectral analysis, Proceedings of the Royal Society of London Series A, 459, 2317-2345 (2003)). Die Phase $\psi_j(t)$ kann auch mittels Wavelet-Analyse bestimmt werden.

Eine Phasenrücksetzung liegt vor, wenn die Phase $\psi_j(t)$ durch einen Reiz (mit Reiz-Beginn bei t = 0) nach einer bestimmten Zeit auf einen Vorzugswert gesetzt wird. D. h., $\{\psi_j(\tau_k + t)\}_{k=1,...,l}$, die von den I Reizantworten gewonnene Verteilung der Werte der Phase $\psi_j(t)$ hat zur Zeit t (relativ zum Burst-Beginn bei t = 0) einen Häufungswert. Dem Fachmann sind unterschiedliche Methoden bekannt, mit denen sich nachweisen lässt, dass eine Verteilung einen Häufungswert (also einen Peak) hat. Eine gebräuchliche Methode ist die Bestimmung des Phasenrücksetzungsindex p(t) mittels zirkulärem Mittelwert:

$$\rho(t) = \left| \frac{1}{l} \sum_{k=1}^{l} \exp\left[i\psi_j(\tau_k + t)\right] \right| \qquad (2)$$

Eine Phasenrücksetzung liegt vor, wenn p(t) z. B. das Maximum oder die 99te Perzentile der prä-Stimulus-Verteilung von p(t) (an einem Zeitpunkt oder innerhalb eines kleinen Zeitfensters von z. B. 20 ms Breite) überschreitet.

In der Praxis hat sich die Analyse mit den gemittelten Antworten $\overline{x}_j(t)$ als ausreichend bewährt.

Für einen als Pulszug ausgestalteten Reiz 22, der eine Phasenrücksetzung bewirken soll, müssen die Dauer der Einzelpulse, die Amplitude der Einzelpulse, die Frequenz, mit der die Pulse im Pulszug periodisch wiederholt werden, und die Anzahl der Pulse im Pulszug bestimmt werden. Für den jeweiligen Patienten und Stimulationsort lassen sich die Reizparameter, die zu einer Phasenrücksetzung der neuronalen Aktivität führen, finden, indem typischerweise drei der vorstehend genannten Parameter fest gewählt werden und ein Parameter variiert wird.

**Patentansprüche**

1. Vorrichtung (1; 2) zur Stimulation von Neuronen, umfassend

   - eine in den Körper eines Patienten implantierbare Stimulationseinheit (11) mit einer Mehrzahl von Stimulationselementen (12) zur Stimulation von Neuronen in einem Zielareal des Gehirns und/oder Rückenmarks des Patienten mit Reizen (22), und
   - eine Steuereinheit (10), welche die Stimulationseinheit (11) derart ansteuert, dass mehrere Gruppen von Stimulationselementen (12) jeweils die Reize (22) erzeugen, wobei
   - die mehreren Gruppen jeweils eine Mehrzahl der Stimulationselemente (12) der Stimulationseinheit (11) umfassen,
   - eine erste Gruppe und eine zweite Gruppe der mehreren Gruppen jeweils Sequenzen von Reizen (22) repetitiv in einem jeweiligen Zeitraster, das aus aufeinanderfolgenden Zyklen besteht, erzeugen,
   - sich die von der ersten Gruppe erzeugten Sequenzen von Reizen (22) von den von der zweiten Gruppe erzeugten Sequenzen von Reizen (22) in der Anzahl nacheinander generierter Sequenzen, nach denen die Reihenfolge, in welcher die Stimulationselemente (12) innerhalb einer Sequenz die Reize (22) erzeugen, variiert wird, und/oder in der Dauer der jeweiligen Zyklen unterscheiden, und
   - die Steuereinheit (10) die Stimulationselemente (12) der ersten Gruppe während eines ersten Zeitintervalls und eines dem ersten Zeitintervall nachfolgenden zweiten Zeitintervalls in unterschiedlichen Stimulationsmodi betreibt, wobei
   - die Steuereinheit (10) die Stimulationselemente (12) während des ersten Zeitintervalls in einem ersten Stimulationsmodus derart ansteuert, dass die Reihenfolge, in welcher die Stimulationselemente (12) innerhalb einer Sequenz die Reize (22) erzeugen, für höchstens 5 nacheinander generierte Sequenzen konstant ist und danach variiert wird, und
   - die Steuereinheit (10) die Stimulationselemente (12) während des zweiten Zeitintervalls in einem zweiten Stimulationsmodus derart ansteuert, dass die Stimulationselemente (12) die Reihenfolge, in welcher die Stimulationselemente (12) innerhalb einer Sequenz die Reize (22) erzeugen, für mindestens 25 nacheinander generierte Sequenzen konstant ist und danach variiert wird,
   - wobei die Stärke der Reize (22) in dem ersten Stimulationsmodus kleiner oder gleich einer vorgegebenen Reizstärke ist und die Stärke der Reize (22) in dem zweiten Stimulationsmodus mindestens das 1,3-fache der vorgegebenen Reizstärke beträgt.

2. Vorrichtung (1; 2) zur Stimulation von Neuronen, umfassend

   - eine in den Körper eines Patienten implantierbare Stimulationseinheit (11) mit einer Mehrzahl von Stimulationselementen (12) zur Stimulation von Neuronen in einem Zielareal des Gehirns und/oder Rückenmarks des Patienten mit Reizen (22), und
   - eine Steuereinheit (10), welche die Stimulationseinheit (11) derart ansteuert, dass mehrere Gruppen von Stimulationselementen (12) jeweils die Reize (22) erzeugen, wobei
   - die mehreren Gruppen jeweils eine Mehrzahl der Stimulationselemente (12) der Stimulationseinheit (11) umfassen,
   - eine erste Gruppe und eine zweite Gruppe der mehreren Gruppen jeweils Sequenzen von Reizen (22) repetitiv in einem jeweiligen Zeitraster, das aus aufeinanderfolgenden Zyklen besteht, erzeugen, und
- sich die von der ersten Gruppe erzeugten Sequenzen von Reizen (22) von den von der zweiten Gruppe erzeugten Sequenzen von Reizen (22) in der Anzahl nacheinander generierter Sequenzen, nach denen die Reihenfolge, in welcher die Stimulationselemente (12) innerhalb einer Sequenz die Reize (22) erzeugen, variiert wird, und/oder in der Dauer der jeweiligen Zyklen unterscheiden,
- wobei die Steuereinheit (10) die Stimulationselemente (12) der zweiten Gruppe derart ansteuert, dass die Reihenfolge, in welcher die Stimulationselemente (12) innerhalb einer Sequenz die Reize (22) erzeugen, für mindestens 20 nacheinander generierte Sequenzen konstant ist und danach variiert wird.

3. Vorrichtung (1; 2) nach Anspruch 2, wobei die Reize (22) dazu ausgelegt sind, bei einer Verabreichung an den Patienten eine krankhaft synchrone und oszillatorische Aktivität von Neuronen zu desynchronisieren, und/oder wobei die von den Stimulationselementen (12) erzeugten Reize (22) derart ausgestaltet sind, dass die Phase der neuronalen Aktivität der stimulierten Neuronen zurückgesetzt wird.

4. Vorrichtung (1; 2) nach einem der Ansprüche 2 bis 3, wobei die erste Gruppe und die zweite Gruppe jeweils innerhalb eines jeweiligen Zyklus entweder genau eine Sequenz von Reizen (22) oder keine Reize erzeugen.

5. Vorrichtung (1; 2) nach einem der Ansprüche 2 bis 4, wobei jedes der Stimulationselemente (12) der ersten und der zweiten Gruppe innerhalb einer jeweiligen Sequenz von Reizen (22) nicht mehr als genau einen Reiz (22) erzeugt.

6. Vorrichtung (1; 2) nach einem der Ansprüche 2 bis 5, wobei jede der Gruppen eine oder mehrere Untergruppen aufweist und die Untergruppen jeweils mindestens eines der zu der jeweiligen Gruppe gehörenden Stimulationselemente (12) umfassen, wobei insbesondere die Stimulationselemente (12) einer jeweiligen Untergruppe zeitgleich die gleichen Reize (22) erzeugen und wobei insbesondere die Vorrichtung (1; 2) ein Anzahl von unabhängigen Stromquellen aufweist, die größer oder gleich der Anzahl der zur Stimulation verwendeten Untergruppen ist.

7. Vorrichtung (1; 2) nach einem der Ansprüche 2 bis 6, wobei das Zielareal mehrere Teilbereiche umfasst und die mehreren Gruppen von Stimulationselementen (12) jeweils einen Teilbereich des Zielareals mit den Reizen (22) stimulieren, ferner umfassend eine Messeinheit (13) zum Aufnehmen von Messsignalen (23), die eine neuronale Aktivität der mit den Reizen (22) stimulierten Neuronen wiedergeben, wobei die Steuereinheit (10) anhand der Messsignale (23) für jeden der von der ersten und der zweiten Gruppe stimulierten Teilbereiche des Zielareals eine dominante Frequenz einer synchronen und oszillatorischen neuronalen Aktivität ermittelt, wobei die Steuereinheit (10) bevorzugt derart ausgestattet ist, dass sie die Dauer der Zyklen für jede von der ersten und zweiten Gruppe an das Inverse der für den jeweiligen Teilbereich ermittelten dominanten Frequenz der synchronen und oszillatorischen neuronalen Aktivität anpasst.

8. Software zum Ausführen in einem Datenverarbeitungssystem, wobei die Software

- Steuersignale zum Ansteuern einer in den Körper eines Patienten implantierten Stimulationseinheit (11) erzeugt, wobei die Stimulationseinheit (11) eine Mehrzahl von Stimulationselementen (12) zur Stimulation von Neuronen in einem Zielareal des Gehirns und/oder Rückenmarks des Patienten mit Reizen (22) umfasst, wobei die Steuersignale die Stimulationseinheit (11) derart ansteuern, dass
- mehrere Gruppen von Stimulationselementen (12) jeweils die Reize (22) erzeugen, wobei
- die Gruppen jeweils eine Mehrzahl der Stimulationselemente (12) der Stimulationseinheit (11) umfassen,
- eine erste Gruppe und eine zweite Gruppe der mehreren Gruppen jeweils Sequenzen von Reizen (22) repetitiv in einem jeweiligen Zeitraster, das aus aufeinanderfolgenden Zyklen besteht, erzeugen,
- sich die von der ersten Gruppe erzeugten Sequenzen von Reizen (22) von den von der zweiten Gruppe erzeugten Sequenzen von Reizen (22) in der Anzahl nacheinander generierter Sequenzen, nach denen die Reihenfolge, in welcher die Stimulationselemente (12) innerhalb einer Sequenz die Reize (22) erzeugen, variiert wird, und/oder in der Dauer der jeweiligen Zyklen unterscheiden, und
- die Stimulationselemente (12) der ersten Gruppe während eines ersten Zeitintervalls und eines dem ersten Zeitintervall nachfolgenden zweiten Zeitintervalls in unterschiedlichen Stimulationsmodi betrieben werden, wobei
- die Stimulationselemente (12) während des ersten Zeitintervalls in einem ersten Stimulationsmodus derart angesteuert werden, dass die Reihenfolge, in welcher die Stimulationselemente (12) innerhalb einer Sequenz die Reize (22) erzeugen, für höchstens 5 nacheinander generierte Sequenzen konstant ist und danach variiert wird, und
- die Stimulationselemente (12) während des zweiten Zeitintervalls in einem zweiten Stimulationsmodus derart angesteuert werden, dass die Stimulationselemente (12) die Reihenfolge, in welcher die Stimulationselemente (12) innerhalb einer Sequenz die Reize (22) erzeugen, für mindestens 25 nacheinander generierte Sequenzen konstant ist und danach variiert wird,

- wobei die Stärke der Reize (22) in dem ersten Stimulationsmodus kleiner oder gleich einer vorgegebenen Reizstärke ist und die Stärke der Reize (22) in dem zweiten Stimulationsmodus mindestens das 1,3-fache der vorgegebenen Reizstärke beträgt.

9. Software zum Ausführen in einem Datenverarbeitungssystem, wobei die Software

- Steuersignale zum Ansteuern einer in den Körper eines Patienten implantierten Stimulationseinheit (11) erzeugt, wobei die Stimulationseinheit (11) eine Mehrzahl von Stimulationselementen (12) zur Stimulation von Neuronen in einem Zielareal des Gehirns und/oder Rückenmarks des Patienten mit Reizen (22) umfasst, wobei die Steuersignale die Stimulationseinheit (11) derart ansteuern, dass

- mehrere Gruppen von Stimulationselementen (12) jeweils die Reize (22) erzeugen, wobei
- die Gruppen jeweils eine Mehrzahl der Stimulationselemente (12) der Stimulationseinheit (11) umfassen,
- eine erste Gruppe und eine zweite Gruppe der mehreren Gruppen jeweils Sequenzen von Reizen (22) repetitiv in einem jeweiligen Zeitraster, das aus aufeinanderfolgenden Zyklen besteht, erzeugen,
- sich die von der ersten Gruppe erzeugten Sequenzen von Reizen (22) von den von der zweiten Gruppe erzeugten Sequenzen von Reizen (22) in der Anzahl nacheinander generierter Sequenzen, nach denen die Reihenfolge, in welcher die Stimulationselemente (12) innerhalb einer Sequenz die Reize (22) erzeugen, variiert wird, und/oder in der Dauer der jeweiligen Zyklen unterscheiden, und
- die Stimulationselemente (12) der zweiten Gruppe derart angesteuert werden, dass die Reihenfolge, in welcher die Stimulationselemente (12) innerhalb einer Sequenz die Reize (22) erzeugen, für mindestens 20 nacheinander generierte Sequenzen konstant ist und danach variiert wird.

10. Vorrichtung (1; 2) zur Stimulation von Neuronen, umfassend

- eine in den Körper eines Patienten implantierbare Stimulationseinheit (11) mit einer Mehrzahl von Stimulationselementen (12) zur Stimulation von Neuronen in einem Zielareal des Gehirns und/oder Rückenmarks des Patienten mit Reizen (22), und
- eine Steuereinheit (10), welche die Stimulationseinheit (11) derart ansteuert, dass mehrere Gruppen von Stimulationselementen (12) jeweils die Reize (22) erzeugen, wobei
- die mehreren Gruppen jeweils eine Mehrzahl der Stimulationselemente (12) der Stimulationseinheit (11) umfassen,
- eine erste Gruppe der mehreren Gruppen dauerhaft einen periodischen Pulszug erzeugt, in welchem die Pulse mit einer Frequenz von mindestens 100 Hz wiederholt werden,
- eine zweite Gruppe der mehreren Gruppen Sequenzen von Reizen (22) repetitiv erzeugt, wobei die von der zweiten Gruppe erzeugten Reize (22) derart ausgestaltet sind, dass die Phase der neuronalen Aktivität der mit den Reizen (22) stimulierten Neuronen zurückgesetzt wird, und
- die erste Gruppe den periodischen Pulszug zumindest während des Zeitraums dauerhaft erzeugt, während dem die zweite Gruppe die Sequenzen von Reizen (22) repetitiv erzeugt.

11. Vorrichtung (1; 2) nach Anspruch 10, wobei

- die Steuereinheit (10) die Stimulationselemente (12) der zweiten Gruppe während eines ersten Zeitintervalls und eines dem ersten Zeitintervall nachfolgenden zweiten Zeitintervalls in unterschiedlichen Stimulationsmodi betreibt, wobei
- die Steuereinheit (10) die Stimulationselemente (12) während des ersten Zeitintervalls in einem ersten Stimulationsmodus derart ansteuert, dass die Reihenfolge, in welcher die Stimulationselemente (12) innerhalb einer Sequenz die Reize (22) erzeugen, für höchstens 5 nacheinander generierte Sequenzen konstant ist und danach variiert wird, und
- die Steuereinheit (10) die Stimulationselemente (12) während des zweiten Zeitintervalls in einem zweiten Stimulationsmodus derart ansteuert, dass die Stimulationselemente (12) die Reihenfolge, in welcher die Stimulationselemente (12) innerhalb einer Sequenz die Reize (22) erzeugen, für mindestens 25 nacheinander generierte Sequenzen konstant ist und danach variiert wird.

12. Vorrichtung (1; 2) nach Anspruch 11, wobei die Stärke der Reize (22) in dem ersten Stimulationsmodus kleiner oder gleich einer vorgegebenen Reizstärke ist und die Stärke der Reize (22) in dem zweiten Stimulationsmodus mindestens das 1,3-fache der vorgegebenen Reizstärke beträgt.

13. Vorrichtung (1; 2) nach einem der Ansprüche 10 bis 12, wobei die Steuereinheit (10) die Stimulationseinheit (11) derart ansteuert, dass nach einer vorgegebenen Zeitdauer die Erzeugung des periodischen Pulszugs durch die erste Gruppe beendet wird und anschließend eine Gruppe der mehreren Gruppen, die zumindest einige der zu der ersten Gruppe gehörenden Stimulationselemente (12) umfasst, Sequenzen von Reizen (22) repetitiv erzeugt, die derart ausgestaltet sind, dass die Phase der neuronalen Aktivität der mit den Reizen (22) stimulierten Neuronen zurückgesetzt wird.

14. Vorrichtung (2) nach einem der Ansprüche 10 bis 12, umfassend eine Messeinheit (13) zum Aufnehmen von Messsignalen (23), die eine neuronale Aktivität der mit den Reizen (22) stimulierten Neuronen wiedergeben, wobei, wenn die Steuereinheit (10) anhand der Messsignale (23) feststellt, dass ein Synchronisationsgrad der durch die zweite Gruppe stimulierten Neuronen durch die Applikation der Sequenzen von Reizen (22) um mindestens einen vorgegebenen Schwellwert reduziert wurde, die Steuereinheit (10) die Erzeugung des periodischen Pulszugs durch die erste Gruppe beendet und anschließend eine dritte Gruppe der mehreren Gruppen, die zumindest einige der zu der ersten Gruppe gehörenden Stimulationselemente (12) umfasst, derart ansteuert, dass die dritte Gruppe Sequenzen von Reizen (22) erzeugt, die derart ausgestaltet sind, dass die Phase der neuronalen Aktivität der mit den Reizen (22) stimulierten Neuronen zurückgesetzt wird.

15. Software zum Ausführen in einem Datenverarbeitungssystem, wobei die Soft ware

- Steuersignale zum Ansteuern einer in den Körper eines Patienten implantierten Stimulationseinheit (11) erzeugt, wobei die Stimulationseinheit (11) eine Mehrzahl von Stimulationselementen (12) zur Stimulation von Neuronen in einem Zielareal des Gehirns und/oder Rückenmarks des Patienten mit Reizen (22) umfasst, wobei die Steuersignale die Stimulationseinheit (11) derart ansteuern, dass
- mehrere Gruppen von Stimulationselementen (12) jeweils die Reize (22) erzeugen, wobei
- die Gruppen jeweils eine Mehrzahl der Stimulationselemente (12) der Stimulationseinheit (11) umfassen,
- eine erste Gruppe der mehreren Gruppen dauerhaft einen periodischen Pulszug erzeugt, in welchem die Pulse mit einer Frequenz von mindestens 100 Hz wiederholt werden,
- eine zweite Gruppe der mehreren Gruppen Sequenzen von Reizen (22) repetitiv erzeugt, wobei die von der zweiten Gruppe erzeugten Reize (22) derart ausgestaltet sind, dass die Phase der neuronalen Aktivität der mit den Reizen (22) stimulierten Neuronen zurückgesetzt wird, und
- die erste Gruppe den periodischen Pulszug zumindest während des Zeitraums dauerhaft erzeugt, während dem die zweite Gruppe die Sequenzen von Reizen (22) repetitiv erzeugt.

**Claims**

1. An apparatus (1; 2) for stimulating neurons, comprising

- a stimulation unit (11) implantable into a patient's body, said stimulation unit comprising a plurality of stimulation elements (12) for stimulating with stimuli (22) neurons in a target area of the patient's brain and/or spinal cord, and
- a control unit (10) controlling the stimulation unit (11) such that each of a plurality of groups of stimulation elements (12) generates the stimuli (22), wherein
- each of the plurality of groups comprises a plurality of the stimulation elements (12) of the stimulation unit (11),
- each of a first group and a second group of the plurality of groups repetitively generates sequences of stimuli (22) within a respective time pattern consisting of successive cycles,
- the sequences of stimuli (22) generated by the first group differ from the sequences of stimuli (22) generated by the second group in the number of consecutively generated sequences after which the order in which the stimulation elements (12) generate the stimuli (22) within a sequence is being varied, and/or in the length of the respective cycles, and
- the control unit (10) operates the stimulation elements (12) of the first group in different stimulation modes during a first time interval and a second time interval following the first time interval, wherein
- the control unit (10) controls the stimulation elements (12) in a first stimulation mode during the first time interval such that the order in which the stimulation elements (12) generate the stimuli (22) within a sequence remains constant for up to five consecutively generated sequences and is then being varied, and
- the control unit (10) controls the stimulation elements (12) in a second stimulation mode during the second time interval such that the order in which the stimulation elements (12) generate the stimuli (22) within a sequence remains constant for at least 25 consecutively generated sequences and is then being varied,
- wherein the intensity of the stimuli (22) in the first stimulation mode is smaller than or equal to a predetermined intensity of stimuli, and the intensity of the stimuli (22) in the second stimulation mode is at least 1.3 times the predetermined intensity of stimuli.

2.  An apparatus (1; 2) for stimulating neurons, comprising

- a stimulation unit (11) implantable into a patient's body, said stimulation unit comprising a plurality of stimulation elements (12) for stimulating with stimuli (22) neurons in a target area of the patient's brain and/or spinal cord, and
- a control unit (10) controlling the stimulation unit (11) such that each of a plurality of groups of stimulation elements (12) generates the stimuli (22), wherein
- each of the plurality of groups comprises a plurality of the stimulation elements (12) of the stimulation unit (11),
- each of a first group and a second group of the plurality of groups repetitively generates sequences of stimuli (22) in a respective time pattern consisting of successive cycles, and
- the sequences of stimuli (22) generated by the first group differ from the sequences of stimuli (22) generated by the second group in the number of consecutively generated sequences after which the order in which the stimulation elements (12) generate the stimuli (22) within a sequence is being varied, and/or in the length of the respective cycles,
- wherein the control unit (10) controls the stimulation elements (12) of the second group such that the order in which the stimulation elements (12) generate the stimuli (22) within a sequence remains constant for at least 20 consecutively generated sequences and is then being varied.

3.  The apparatus (1; 2) according to claim 2, wherein the stimuli (22) are configured to desynchronize an abnormally synchronous and oscillatory activity of neurons when being applied to the patient, and/or wherein the stimuli (22) generated by the stimulation elements (12) are configured such that the phase of the neuronal activity of the stimulated neurons is being reset.

4.  The apparatus (1; 2) according to any one of claims 2 to 3, wherein each of the first group and the second group generate either exactly one sequence of stimuli (22) or no stimuli within a respective cycle.

5.  The apparatus (1; 2) according to any one of claims 2 to 4, wherein each of the stimulation elements (12) of the first and second groups does not generate more than exactly one stimulus (22) within a respective sequence of stimuli (22).

6.  The apparatus (1; 2) according to any one of claims 2 to 5, wherein each of the groups comprises one or a plurality of subgroups, and each of the subgroups comprises at least one of the stimulation elements (12) belonging to the respective group, wherein particularly the stimulation elements (12) of a respective subgroup generate equal stimuli (22) at the same time, and wherein particularly the apparatus (1; 2) comprises a number of independent power sources greater than or equal to the number of the subgroups used for stimulation.

7.  The apparatus (1; 2) according to any one of claims 2 to 6, wherein the target area comprises a plurality of partitions, and each of the plurality of groups of stimulation elements (12) stimulate with the stimuli (22) a partition of the target area, further comprising a measurement unit (13) for receiving measurement signals (23) that represent a neuronal activity of the neurons stimulated with the stimuli (22), wherein by means of the measurement signals (23) the control unit (10) determines a dominant frequency of a synchronous and oscillatory neuronal activity for any partition of the target area stimulated by the first and second groups, wherein the control unit (10) is preferably configured such that it adapts the length of the cycles for any one of the first and second groups to the inverse of the dominant frequency of the synchronous and oscillatory neuronal activity determined for the respective partition.

8.  A software for execution in a data processing system, wherein the software

- creates control signals for controlling a stimulation unit (11) implanted into a patient's body, wherein the stimulation unit (11) comprises a plurality of stimulation elements (12) for stimulating with stimuli (22) neurons in a target area of the patient's brain and/or spinal cord, wherein the control signals control the stimulation unit (11) such that
- each of a plurality of groups of stimulation elements (12) generates the stimuli (22),

wherein

- each of the plurality of groups comprises a plurality of the stimulation elements (12) of the stimulation unit (11),
- each of a first group and a second group of the plurality of groups repetitively generates sequences of stimuli (22) within a respective time pattern consisting of successive cycles,
- the sequences of stimuli (22) generated by the first group differ from the sequences of stimuli (22) generated by the second group in the number of consecutively generated sequences after which the order in which the stimulation elements (12) generate the stimuli (22) within a sequence is being varied, and/or in the length of the respective cycles, and
- the stimulation elements (12) of the first group are operated in different stimulation modes during a first time interval and a second time interval following the first time interval, wherein
- during the first time interval in a first stimulation mode the stimulation elements (12) are controlled such that the order in which the stimulation elements (12) generate the stimuli (22) within a sequence remains constant for up to five consecutively generated sequences and is then being varied, and
- during the second time interval in a second stimulation mode the stimulation elements (12) are controlled such that the order in which the stimulation elements (12) generate the stimuli (22) within a sequence remains constant for at least 25 consecutively generated sequences and is then being varied,
- wherein the intensity of the stimuli (22) in the first stimulation mode is smaller than or equal to a predetermined intensity of stimuli, and the intensity of the stimuli (22) in the second stimulation mode is at least 1.3 times the predetermined intensity of stimuli.

9. A software for execution in a data processing system, wherein the software

   - creates control signals for controlling a stimulation unit (11) implanted into a patient's body, wherein the stimulation unit (11) comprises a plurality of stimulation elements (12) for stimulating with stimuli (22) neurons in a target area of the patient's brain and/or spinal cord, wherein the control signals control the stimulation unit (11) such that
   - each of a plurality of groups of stimulation elements (12) generates the stimuli (22),

   wherein

   - each of the plurality of groups comprises a plurality of the stimulation elements (12) of the stimulation unit (11),
   - each of a first group and a second group of the plurality of groups repetitively generates sequences of stimuli (22) within a respective time pattern consisting of successive cycles,
   - the sequences of stimuli (22) generated by the first group differ from the sequences of stimuli (22) generated by the second group in the number of consecutively generated sequences after which the order in which the stimulation elements (12) generate the stimuli (22) within a sequence is being varied, and/or in the length of the respective cycles, and
   - the stimulation elements (12) of the second group are controlled such that the order in which the stimulation elements (12) generate the stimuli (22) within a sequence remains constant for at least 20 consecutively generated sequences and is then being varied.

10. An apparatus (1; 2) for stimulating neurons, comprising

   - a stimulation unit (11) implantable into a patient's body, said stimulation unit comprising a plurality of stimulation elements (12) for stimulating with stimuli (22) neurons in a target area of the patient's brain and/or spinal cord, and
   - a control unit (10) controlling the stimulation unit (11) such that each of a plurality of groups of stimulation elements (12) generates the stimuli (22), wherein
   - each of the plurality of groups comprises a plurality of the stimulation elements (12) of the stimulation unit (11),
   - a first group of the plurality of groups permanently generates a periodical pulse train in which the pulses are repeated at a frequency of at least 100 Hz,
   - a second group of the plurality of groups repetitively generates sequences of stimuli (22), wherein the stimuli (22) generated by the second group are configured such that the phase of the neuronal activity of the neurons stimulated with the stimuli (22) is reset, and
   - the first group permanently generates the periodical pulse train at least during the period in which the second group repetitively generates the sequences of stimuli (22).

11. The apparatus (1; 2) according to claim 10, wherein

   - the control unit (10) operates the stimulation elements (12) of the second group in different stimulation modes during a first time interval and a second time interval following the first time interval, wherein

- the control unit (10) controls the stimulation elements (12) in a first stimulation mode during the first time interval such that the order in which the stimulation elements (12) generate the stimuli (22) within a sequence remains constant for up to five consecutively generated sequences and is then being varied, and
- the control unit (10) controls the stimulation elements (12) in a second stimulation mode during the second time interval such that the order in which the stimulation elements (12) generate the stimuli (22) within a sequence remains constant for at least 25 consecutively generated sequences and is then being varied.

12. The apparatus (1; 2) according to claim 11, wherein the intensity of the stimuli (22) in the first stimulation mode is smaller than or equal to a predetermined intensity of stimuli, and the intensity of the stimuli (22) in the second stimulation mode is at least 1.3 times the predetermined intensity of stimuli.

13. The apparatus (1; 2) according to any one of claims 10 to 12, wherein the control unit (10) controls the stimulation unit (11) such that the generation of the periodical pulse train by the first group is finished after a predetermined period and that subsequently a group of the plurality of groups which comprises at least a few of the stimulation elements (12) belonging to the first group repetitively generates sequences of stimuli (22) which are configured such that the phase of the neuronal activity of the neurons stimulated with the stimuli (22) is reset.

14. The apparatus (1; 2) according to any one of claims 10 to 12, comprising a measurement unit (13) for receiving measurement signals (23) which represent a neuronal activity of the neurons stimulated with the stimuli (22), wherein, when the control unit (10) determines by means of the measurement signals (23) that a degree of synchronization of the neurons stimulated by the second group has been reduced by at least one predetermined threshold value by application of the sequences of stimuli (22), the control unit (10) finishes the generation of the periodical pulse train by the first group and then controls a third group of the plurality of groups which comprises at least a few of the stimulation elements (12) belonging to the first group such that the third group generates sequences of stimuli (22) which are configured such that the phase of the neuronal activity of the neurons stimulated with the stimuli (22) is reset.

15. A software for execution in a data processing system, wherein the software

- generates control signals for controlling a stimulation unit (11) implanted into a patient's body, wherein the stimulation unit (11) comprises a plurality of stimulation elements (12) for stimulating with stimuli (22) neurons in a target area of the patient's brain and/or spinal cord, wherein the control signals control the stimulation unit (11) such that
- each of a plurality of groups of stimulation elements (12) generates the stimuli (22),

wherein

- each of the groups comprises a plurality of the stimulation elements (12) of the stimulation unit (11),
- a first group of the plurality of groups permanently generates a periodical pulse train in which the pulses are repeated at a frequency of at least 100 Hz,
- a second group of the plurality of groups repetitively generates sequences of stimuli (22), wherein the stimuli (22) generated by the second group are configured such that the phase of the neuronal activity of the neurons stimulated with the stimuli (22) is reset, and
- the first group permanently generates the periodical pulse train at least during the period in which the second group repetitively generates the sequences of stimuli (22).

**Revendications**

1. Dispositif (1 ; 2) pour la stimulation de neurones, comprenant

- une unité de stimulation (11) implantable dans le corps d'un patient avec une multiplicité d'éléments de stimulation (12) pour la stimulation de neurones dans une zone cible du cerveau et/ou de la moelle épinière du patient avec des excitations (22), et
- une unité de commande (10), qui commande l'unité de stimulation (11) de telle manière que plusieurs groupes d'éléments de stimulation (12) produisent chacun les excitations (22), où
- les plusieurs groupes comprennent chacun une multiplicité des éléments de stimulation (12) de l'unité de stimulation (11),

- un premier groupe et un second groupe des plusieurs groupes produisent chacun des séquences d'excitations (22) de manière répétitive dans une trame temporelle correspondante, qui consiste en cycles qui se succèdent,
- les séquences d'excitations (22) produites par le premier groupe diffèrent des séquences d'excitations (22) produites par le second groupe dans le nombre de séquences générées les unes après les autres, après lesquelles la succession dans laquelle les éléments de stimulation (12) produisent les excitations (22) à l'intérieur d'une séquence est amenée à varier, et/ou dans la durée des cycles correspondants, et
- l'unité de commande (10) actionne les éléments de stimulation (12) du premier groupe pendant un premier intervalle de temps et un second intervalle de temps suivant le premier intervalle de temps dans des modes de stimulation différents, où
- l'unité de commande (10) commande les éléments de stimulation (12) pendant le premier intervalle de temps dans un premier mode de stimulation de telle manière que la succession dans laquelle les éléments de stimulation (12) produisent les excitations (22) à l'intérieur d'une séquence est constante pour au plus 5 séquences générées les unes après les autres et est ensuite amenée à varier, et
- l'unité de commande (10) commande les éléments de stimulation (12) pendant le second intervalle de temps dans un second mode de stimulation de telle manière que la succession dans laquelle les éléments de stimulation (12) produisent les excitations (22) à l'intérieur d'une séquence est constante pour au moins 25 séquences générées les unes après les autres et est ensuite amenée à varier,
- où l'intensité des excitations (22) dans le premier mode de stimulation est inférieure ou égale à une intensité d'excitation prédéfinie et l'intensité des excitations (22) dans le second mode de stimulation est au moins 1,3 fois l'intensité d'excitation prédéfinie.

2. Dispositif (1 ; 2) pour la stimulation de neurones, comprenant

- une unité de stimulation (11) implantable dans le corps d'un patient avec une multiplicité d'éléments de stimulation (12) pour la stimulation de neurones dans une zone cible du cerveau et/ou de la moelle épinière du patient avec des excitations (22), et
- une unité de commande (10), qui commande l'unité de stimulation (11) de telle manière que plusieurs groupes d'éléments de stimulation (12) produisent chacun les excitations (22), où
- les plusieurs groupes comprennent chacun une multiplicité des éléments de stimulation (12) de l'unité de stimulation (11),
- un premier groupe et un second groupe des plusieurs groupes produisent chacun des séquences d'excitations (22) de manière répétitive dans une trame temporelle correspondante, qui consiste en cycles qui se succèdent, et
- les séquences d'excitations (22) produites par le premier groupe diffèrent des séquences d'excitations (22) produites par le second groupe dans le nombre de séquences générées les unes après les autres, après lesquelles la succession dans laquelle les éléments de stimulation (12) produisent les excitations (22) à l'intérieur d'une séquence est amenée à varier, et/ou dans la durée des cycles correspondants,
- où l'unité de commande (10) commande les éléments de stimulation (12) du second groupe de telle manière que la succession dans laquelle les éléments de stimulation (12) produisent les excitations (22) à l'intérieur d'une séquence est constante pour au moins 20 séquences générées les unes après les autres et est ensuite amenée à varier.

3. Dispositif (1 ; 2) selon la revendication 2, où les excitations (22) sont prévues pour, lors d'une administration au patient, désynchroniser une activité de neurones synchrone et oscillatoire pathologique, et/ou bien où les excitations (22) produites par les éléments de stimulation (12) sont agencées de telle manière que la phase de l'activité neuronale des neurones stimulés est réinitialisée.

4. Dispositif (1 ; 2) selon l'une des revendications 2 à 3, où le premier groupe et le second groupe produisent chacun à l'intérieur d'un cycle correspondant exactement une séquence d'excitations (22) ou ne produisent chacun aucune excitation.

5. Dispositif (1 ; 2) selon l'une des revendications 2 à 4, où chacun des éléments de stimulation (12) du premier et du second groupe ne produit pas plus d'exactement une excitation (22) à l'intérieur d'une séquence de stimulations (22) correspondante.

6. Dispositif (1 ; 2) selon l'une des revendications 2 à 5, où chacun des groupes présente un ou plusieurs sous-groupes et les sous-groupes comprennent chacun au moins l'un des éléments de stimulation (12) appartenant au groupe correspondant, où en particulier les éléments de stimulation (12) d'un sous-groupe correspondant produisent simultanément les mêmes excitations (22) et où en particulier le dispositif (1 ; 2) présente un nombre de sources de courant indépendantes qui est supérieur ou égal au nombre des sous-groupes utilisés pour la stimulation.

7. Dispositif (1 ; 2) selon l'une des revendications 2 à 6, où la zone cible comprend plusieurs domaines partiels et les plusieurs groupes d'éléments de stimulation (12) stimulent chacun un domaine partiel de la zone cible avec les excitations (22), comprenant en outre une unité de mesure (13) pour la réception de signaux de mesure (23) qui reflètent une activité neuronale des neurones stimulés avec les excitations (22), où l'unité de commande (10) détermine à l'aide des signaux de mesure (23) pour chacun des domaines partiels de la zone cible stimulés par le premier et le second groupe une fréquence dominante d'une activité neuronale synchrone et oscillatoire, où l'unité de commande (10) est de préférence agencée de telle manière qu'elle adapte la durée des cycles pour chacun du premier et du second groupe à l'inverse de la fréquence dominante de l'activité neuronale synchrone et oscillatoire déterminée pour le domaine partiel correspondant.

8. Logiciel pour la mise en œuvre dans un système de traitement de données, où le logiciel

   - produit des signaux de commande pour la commande d'une unité de stimulation (11) implantée dans le corps d'un patient, où l'unité de stimulation (11) comprend une multiplicité d'éléments de stimulation (12) pour la stimulation de neurones dans une zone cible du cerveau et/ou de la moelle épinière du patient avec des excitations (22), où les signaux de commande commandent l'unité de stimulation (11) de telle manière que
   - plusieurs groupes d'éléments de stimulation (12) produisent chacun les excitations (22), où
   - les groupes comprennent chacun une multiplicité des éléments de stimulation (12) de l'unité de stimulation (11),
   - un premier groupe et un second groupe des plusieurs groupes produisent chacun des séquences d'excitations (22) de manière répétitive dans une trame temporelle correspondante, qui consiste en cycles qui se succèdent,
   - les séquences d'excitations (22) produites par le premier groupe diffèrent des séquences d'excitations (22) produites par le second groupe dans le nombre de séquences générées les unes après les autres, après lesquelles la succession dans laquelle les éléments de stimulation (12) produisent les excitations (22) à l'intérieur d'une séquence est amenée à varier, et/ou dans la durée des cycles correspondants, et
   - les éléments de stimulation (12) du premier groupe sont actionnés pendant un premier intervalle de temps et un second intervalle de temps suivant le premier intervalle de temps dans des modes de stimulation différents, où
   - les éléments de stimulation (12) sont commandés pendant le premier intervalle de temps dans un premier mode de stimulation de telle manière que la succession dans laquelle les éléments de stimulation (12) produisent les excitations (22) à l'intérieur d'une séquence est constante pour au plus 5 séquences générées les unes après les autres et est ensuite amenée à varier, et
   - les éléments de stimulation (12) sont commandés pendant le second intervalle de temps dans un second mode de stimulation de telle manière que la succession dans laquelle les éléments de stimulation (12) produisent les excitations (22) à l'intérieur d'une séquence est constante pour au moins 25 séquences générées les unes après les autres et est ensuite amenée à varier,
   - où l'intensité des excitations (22) dans le premier mode de stimulation est inférieure ou égale à une intensité d'excitation prédéfinie et l'intensité des excitations (22) dans le second mode de stimulation est au moins 1,3 fois l'intensité d'excitation prédéfinie.

9. Logiciel pour la mise en œuvre dans un système de traitement de données, où le logiciel

   - produit des signaux de commande pour la commande d'une unité de stimulation (11) implantée dans le corps d'un patient, où l'unité de stimulation (11) comprend une multiplicité d'éléments de stimulation (12) pour la stimulation de neurones dans une zone cible du cerveau et/ou de la moelle épinière du patient avec des excitations (22), où les signaux de commande commandent l'unité de stimulation (11) de telle manière que
   - plusieurs groupes d'éléments de stimulation (12) produisent chacun les excitations (22), où
   - les groupes comprennent chacun une multiplicité des éléments de stimulation (12) de l'unité de stimulation (11),
   - un premier groupe et un second groupe des plusieurs groupes produisent chacun des séquences d'excitations (22) de manière répétitive dans une trame temporelle correspondante, qui consiste en cycles qui se succèdent,
   - les séquences d'excitations (22) produites par le premier groupe diffèrent des séquences d'excitations (22) produites par le second groupe dans le nombre de séquences générées les unes après les autres, après lesquelles la succession dans laquelle les éléments de stimulation (12) produisent les excitations (22) à l'intérieur d'une séquence est amenée à varier, et/ou dans la durée des cycles correspondants, et
   - les éléments de stimulation (12) du second groupe sont commandés de telle manière que la succession dans laquelle les éléments de stimulation (12) produisent les excitations (22) à l'intérieur d'une séquence est constante pour au moins 20 séquences générées les unes après les autres et est ensuite amenée à varier.

**10.** Dispositif (1 ; 2) pour la stimulation de neurones, comprenant

- une unité de stimulation (11) implantable dans le corps d'un patient avec une multiplicité d'éléments de stimulation (12) pour la stimulation de neurones dans une zone cible du cerveau et/ou de la moelle épinière du patient avec des excitations (22), et
- une unité de commande (10), qui commande l'unité de stimulation (11) de telle manière que plusieurs groupes d'éléments de stimulation (12) produisent chacun les excitations (22), où
- les plusieurs groupes comprennent chacun une multiplicité des éléments de stimulation (12) de l'unité de stimulation (11),
- un premier groupe des plusieurs groupes produit de manière durable un train d'impulsions périodique dans lequel les impulsions sont répétées avec une fréquence d'au moins 100 Hz,
- un second groupe des plusieurs groupes produit de manière répétitive des séquences d'excitations (22), où les excitations (22) produites par le second groupe sont agencées de telle manière que la phase de l'activité neuronale des neurones stimulés avec les excitations (22) est réinitialisée, et
- le premier groupe produit le train d'impulsions périodique de manière durable au moins pendant la durée au cours de laquelle le second groupe produit les séquences d'excitations (22) de manière répétitive.

**11.** Dispositif (1 ; 2) selon la revendication 10, où

- l'unité de commande (10) actionne les éléments de stimulation (12) du second groupe pendant un premier intervalle de temps et un second intervalle de temps suivant le premier intervalle de temps dans des modes de stimulation différents, où
- l'unité de commande (10) commande les éléments de stimulation (12) pendant le premier intervalle de temps dans un premier mode de stimulation de telle manière que la succession dans laquelle les éléments de stimulation (12) produisent les excitations (22) à l'intérieur d'une séquence est constante pour au plus 5 séquences générées les unes après les autres et est ensuite amenée à varier, et
- l'unité de commande (10) commande les éléments de stimulation (12) pendant le second intervalle de temps dans un second mode de stimulation de telle manière que la succession dans laquelle les éléments de stimulation (12) produisent les excitations (22) à l'intérieur d'une séquence est constante pour au moins 25 séquences générées les unes après les autres et est ensuite amenée à varier.

**12.** Dispositif (1 ; 2) selon la revendication 11, où l'intensité des excitations (22) dans le premier mode de stimulation est inférieure ou égale à une intensité d'excitation préétablie et l'intensité des excitations (22) dans le second mode de stimulation est au moins 1,3 fois l'intensité d'excitation préétablie.

**13.** Dispositif (1 ; 2) selon l'une des revendications 10 à 12, où l'unité de commande (10) commande l'unité de stimulation (11) de telle manière qu'après une durée préétablie la production du train d'impulsions périodique par le premier groupe est arrêtée et ensuite un groupe des plusieurs groupes, qui comprend au moins quelques-uns des éléments de stimulation (12) appartenant au premier groupe, produit des séquences d'excitations (22) de manière répétitive, qui sont agencées de telle manière que la phase de l'activité neuronale des neurones stimulés avec les excitations (22) est réinitialisée.

**14.** Dispositif (1 ; 2) selon l'une des revendications 10 à 12, comprenant une unité de mesure (13) pour la réception de signaux de mesure (23) qui reflètent une activité neuronale des neurones stimulés avec les excitations (22), où, quand l'unité de commande (10) établit à l'aide des signaux de mesure (23) qu'un degré de synchronisation des neurones stimulés par le second groupe a été réduit par l'application des séquences d'excitations (22) d'au moins une valeur seuil préétablie, l'unité de commande (10) arrête la production du train d'impulsions périodique par le premier groupe et commande ensuite un troisième groupe des plusieurs groupes, qui comprend au moins quelques-uns des éléments de stimulation (12) appartenant au premier groupe, de telle manière que le troisième groupe produit des séquences d'excitations (22) qui sont agencées de telle manière que la phase de l'activité neuronale des neurones stimulés avec les excitations (22) est réinitialisée.

**15.** Logiciel pour la mise en œuvre dans un système de traitement de données, où le logiciel

- produit des signaux de commande pour la commande d'une unité de stimulation (11) implantée dans le corps d'un patient, où l'unité de stimulation (11) comprend une multiplicité d'éléments de stimulation (12) pour la stimulation de neurones dans une zone cible du cerveau et/ou de la moelle épinière du patient avec des excitations (22), où les signaux de commande commandent l'unité de stimulation (11) de telle manière que

- plusieurs groupes d'éléments de stimulation (12) produisent chacun les excitations (22), où
- les groupes comprennent chacun une multiplicité des éléments de stimulation (12) de l'unité de stimulation (11),
- un premier groupe des plusieurs groupes produit de manière durable un train d'impulsions périodique dans lequel les impulsions sont répétées avec une fréquence d'au moins 100 Hz,
- un second groupe des plusieurs groupes produit de manière répétitive des séquences d'excitations (22), où les excitations (22) produites par le second groupe sont agencées de telle manière que la phase de l'activité neuronale des neurones stimulés avec les excitations (22) est réinitialisée, et
- le premier groupe produit le train d'impulsions périodique de manière durable au moins pendant la durée au cours de laquelle le second groupe produit les séquences d'excitations (22) de manière répétitive.

## Fig.1

## Fig.2

## Fig.3

## Fig.4

Fig.5

Fig.6

## Fig.7

## Fig.8

Gruppe 1

Gruppe 2

Gruppe 3

# Fig.9

# Fig.10

## Fig.11

Gruppe 1

22

1_1
1_2
1_3

$T_{stim\_1}$

$T_{stim\_1}/3$

Gruppe 2

48

2_1
2_2
2_3

t

22

Gruppe 3

3_1
3_2
3_3
3_4

t

$T_{stim\_3}$

$T_{stim\_3}/4$

## Fig.12

50  51

53  52  S2  54

S1

S3

58  57  56  55

S5  S4

## Fig.13

Fig.14

Fig.15

## Fig.16

80

81

2_1

2_2

2_3

2_4

Gruppe 2

1_1

1_2

1_3

Gruppe 1

## Fig.17

80

81

2_1

2_2

2_3

Gruppe 2

1_1

1_2

1_3

Gruppe 1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102009025407 A1
- DE 102012002437 A1
- EP 1944059 A2

**In der Beschreibung aufgeführte Nicht-Patentliteratur**

- **P. TEMPERLI ; J. GHIKA ; J.-G. VILLEMURE ; P. BURKHARD ; J. BOGOUSSLAVSKY ; F. VINGER-HOETS.** How do parkinsonian signs return after discontinuation of subthalamic DBS. *Neurology,* 2003, vol. 60, 78
- **P. A. TASS ; L. QIN ; C. HAUPTMANN ; S. DOVEROS ; E. BEZARD ; T. BORAUD ; W. G. MEISSNER.** Coordinated reset neuromodulation has sustained after-effects in parkinsonian monkeys. *Annals of Neurology,* 2012, vol. 72, 816-820
- **I. ADAMCHIC ; C. HAUPTMANN ; U. B. BARNIKOL ; N. PAWELCYK ; O.V. POPOVYCH ; T. BARNIKOL ; A. SILCHENKO ; J. VOLKMANN ; G. DEUSCHL ; W. MEISSNER.** Coordinated Reset Has Lasting Aftereffects in Patients with Parkinson's Disease. *Movement Disorders,* 2014, vol. 29, 1679
- **H. C. MARTENS ; E. TOADER ; M. M. DECRE et al.** Spatial steering of deep brain stimulation volumes using a novel lead design. *Clinical neurophysiology,* 2011, vol. 122, 558-566
- **J. BUHLMANN ; L. HOFMANN ; P. A. TASS ; C. HAUPTMANN.** Modeling of a segmented electrode for desynchronizing deep brain stimulation. *Frontiers in Neuroengineering,* 2011, vol. 4, 15

- **C. MOREAU ; L. DEFEBVRE ; A. DESTEE et al.** STN-DBS frequency effects on freezing of gait in advanced Parkinson disease. *Neurology,* 2008, vol. 71, 80-84
- **M. JAHANSHAHI ; I. OBESO ; C. BAUNEZ et al.** Parkinson's disease, the subthalamic nucleus, inhibition, and impulsivity. *Movement Disorders,* 2015, vol. 30, 128-140
- **P. A. TASS.** Transmission of stimulus-locked responses in two coupled phase oscillators. *Phys. Rev. E,* 2004, vol. 69, 051909-1, 24
- **P. A. TASS.** Transmission of stimulus-locked responses in two coupled phase oscillators. *Phys. Rev.,* 2004, vol. 69, 051909-1, 24
- **N. E. HUANG et al.** The empirical mode decomposition and the Hilbert spectrum for nonlinear and non-stationary time series analysis. *Proc. R. Soc. A: Math. Phys. Eng. Sci.,* 1998, vol. 454, 903-995
- **N. E. HUANG et al.** A confidence limit for the empirical mode decomposition and Hilbert spectral analysis. *Proceedings of the Royal Society of London Series A,* 2003, vol. 459, 2317-2345